# EUROPEAN PATENT SPECIFICATION

(11) **EP 2 897 608 B1**
(45) Date of publication and mention of the grant of the patent: **22.08.2018**
(21) Application number: 14787478.8
(22) Date of filing: 21.04.2014
(51) Int. Cl.: A61K 31/365, A61K 36/19, A61P 25/28, A61K 31/7028

(54) **TREATMENT OF ALZHEIMER'S AND COGNITIVE IMPAIRMENT WITH ANDROGRAPHOLIDES**
BEHANDLUNG VON MORBUS ALZHEIMER UND KOGNITIVEN STÖRUNGEN MIT ANDROGRAPHOLIDEN
TRAITEMENT DE LA MALADIE D'ALZHEIMER ET D'ATTEINTES COGNITIVES AU MOYEN D'ANDROGRAPHOLIDES

(30) Priority: 22.04.2013 US 201361814445 P
(43) Date of publication of application: 29.07.2015
(73) Proprietor: InnoBioscience, LLC, Bradenton, FL 34209 (US); Inestrosa, Nibaldo, C., Santiago, Region Metropolitana (CL); Hankcke Orozco, Juan, L., Santa Rita, Pirque (CL)
(72) Inventor: INESTROSA, Nibaldo, C., Santiago, Region Metropolitana (CL); HANKCKE OROZCO, Juan, L., Santa Rita, Pirque (CL)
(74) Representative: Teuten, Andrew John
(86) International application number: PCT/US2014/034758
(87) International publication number: WO 2014/176149

(56) References cited:
- WO-A1-2011/086007
- WO-A2-01/51659
- CN-A- 101 371 832
- CN-A- 103 224 492
- US-A1- 2006 063 831
- US-A1- 2008 103 165
- US-A1- 2008 103 196
- US-A1- 2012 301 560
- Jiang Li ET AL: "Effects of andrographolide on cognitive function and hippocampal synaptic plasticity in the rats with vascular dementia", Journal of Community Medicine, 1 February 2014 (2014-02-01), pages 1-3, XP055254910, Retrieved from the Internet: URL:http://en.cnki.com.cn/Article_en/CJFDT otal-SQYX201402001.htm [retrieved on 2016-03-03]

## Description

### INTRODUCTION:

The mechanisms involved in the pathogenic changes triggered in Alzheimer's disease (AD) are not clearly understood, the neuronal dysfunction, which includes synaptic failure associated with loss of synaptic proteins and a reduction in the neuronal survival by accumulation of amyloid β-peptide (Aβ), contributing to the progression of the disease. In fact, the use of natural compounds is an interesting conceptual strategy in the search of drugs with therapeutic potential for the treatment of neurodegenerative disorders. Here, we report that Andrographolide (ANDRO), a labdane diterpene and a major component of *Andrographis paniculata* prevents and reverses the neuropathology in two aged set of mice model of AD (7-month and 12-month old mice respectively). In both sets of transgenic animals, ANDRO recovers spatial memory functions correlated with synaptic plasticity and synaptic proteins. ANDRO decreases *tau* phosphorylation around the oligomeric species and reduces the levels of Aβ changing the ontogeny of Aβ plaques altering the Aβ levels in the hippocampus and cortex in 6 month-old mice. ANDRO increases also field excitatory postsynaptic potentials in the CA1 region of hippocampus slices inhibiting the long-term depression (LTD) by a mechanism that probably involves an inhibition of the glycogen synthase kinase-3β. Our results suggest that ANDRO might be used in a potential preventive and reversible therapy during the progression state of AD.

**Abbreviations:** APP = Amyloid Precursor Protein; PS-1 = Presenilin-1; A = Amyloid -peptide; ANDRO = Andrographolide; PSD-95 = Postsynaptic Density Protein-95; SYP = Synaptophysin; GFAP = Glial Fibrillary Acidic Protein; Glycogen Synthase Kinase-3β = GSK-3β; MWM = Morris Water Maze; LTP = Long-Term Potentiation; LTD = Long-Term Depression.

Alzheimer's disease (AD) is the most common cause of dementia and to date there is no known cure available. The underlying neuropathology of AD includes extracellular deposition of amyloid β-peptide (Aβ), intra-neuronal accumulation of hyperphosphorylated *tau* forms (Mandelkow & Mandelkow 2012; Selkoe et al 2012) as well as synapse dysfunction and neuronal loss (Scheff et al 2007; Selkoe et al 2012; Selkoe 2002; Terry et al 1991). Analysis of AD patients' brains supports the hypothesis that aggregates of Aβ are responsible for synaptic failure (Mucke & Selkoe 2012) and generation of animal models recapitulate the characteristic features of AD, which have great relevance to improve the understanding of this disease and to help develop new therapies (Braidy et al 2012; Garcia-Alloza et al 2006)

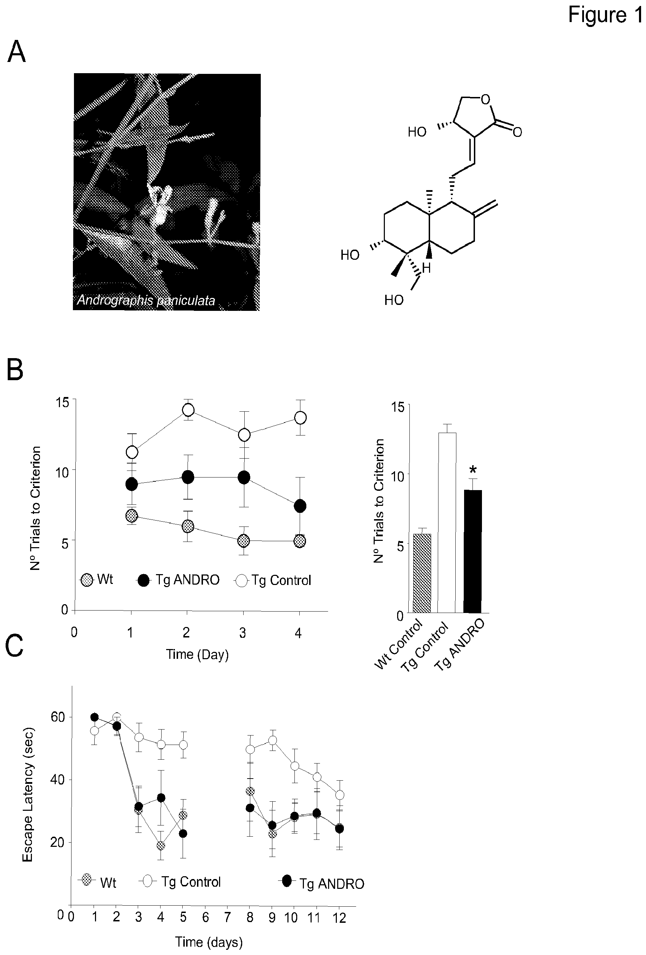

*Andrographis paniculata* is a native plant from Southeast Asian countries. For centuries, it has been used as an official herbal medicine in China for the treatment of a variety of human illnesses (Tang & Eisenbrandt 1992). Previous studies have indicated that andrographolide (ANDRO), a diterpene of the labdane family, is responsible for most of the biological effects of *Andrographis paniculata* (Figure 1A) (Basak et al 1999; Hidalgo et al 2005; Iruretagoyena et al 2005). Recent studies suggest that ANDRO might exert some neuroprotective effects, i.e., against damage induced by dopamine in mesencephalic neuron-glial cultures (Wang et al 2004), oxidative stress induced by nicotine in brain (Das et al 2009) and in cerebral ischemia (Chan et al 2010), through the inhibition of certain pathways dependent on apoptosis, Akt and NF-κB (Lu et al 2011). However, the role of ANDRO in neurodegenerative diseases such as AD has not been investigated.

Here we explored whether ANDRO exhibits beneficial effects in an AD transgenic mouse model with APP and PS-1 mutant transgenes (APP/PS1) (Garcia-Alloza et al 2006). Young and aged transgenic mice were treated with ANDRO and used for behavioral, electrophysiological, biochemical and histochemical analysis. We found a recovery of memory, synaptic functions, long-term potentiation (LTP) and a clear reduction in *tau* phosphorylation in both set of animals. Interestingly, in 7-month old mice we detect a reduction in Aβ species and aggregates. Nonetheless, in 12-month old mice we do not have significant Aβ oligomeric changes in hippocampus. With the idea to find a possible mechanism, we show that ANDRO inhibited the long-term depression (LTD) indicating a synaptic mechanism, which is related to the inhibition of GSK-3β activity (Bradley et al 2012b; Peineau et al 2007). Our results suggest that ANDRO may provide beneficial effects for the treatment of AD at different age levels.

WO2011/086007 (Indena S.P.A.) relates to compositions comprising *Andrographis paniculata* extracts combined with Ginkgo biloba extracts complexed with phospholipids, and their use in the treatment of neurodegenerative disorders, in particular Alzheimer's disease and multiple sclerosis.

### SUMMARY OF THE INVENTION

The invention provides andrographolide for use in a method for the treatment of Alzheimer's disease dementia in a human wherein the andrographolide is administered in a therapeutically effective amount as a daily oral dosage of from 1 to 4 milligrams per kilogram of body weight of a human.

Suitably, the method for the treatment of Alzheimer's disease dementia in a human comprises:
a) Administering to a human a test selected from the group consisting of: the Mini Mental Status Exam (MMSE), the Alzheimer Disease Assessment Scale (ADAS), the Boston Naming Test (BNT) and the Token Test (TT); and then
b) Administering andrographolide to said human, for at least 30 days, a daily dosage effective to preserve or improve cognitive function.

Suitably, the andrographolide is provided in a therapeutically effective amount.

Suitably, the andrographolide is provided as an extract of *Andrographis paniculata,* standardized to contain not less than 50% (w/w) of andrographolide.

Suitably, the andrographolide is pure andrographolide.

### BRIEF DESCRIPTION OF THE DRAWINGS

**Figure 1****: ANDRO recovers the cognitive functions in an AD mice model of different ages.** (**A**) Andrographolide (ANDRO) is a diterpene of the labdane family purified from *Andrographis paniculata.* (**B**) Behavioral performance in the memory flexibility test. APPswe/PS-1 mice from 6 month-old was treated IP with control vehicle solution (Wt, gray circles and Tg Control, white circles) or with ANDRO (Tg ANDRO, black circles). (**C**) Behavioral performance was evaluated by the Escape latency in the classical MWM test. Wt and APPswe/PS-1 mice from 12 month-old were treated with vehicle solution (Wt, gray circles and Tg Control, white circles) or ANDRO (Tg ANDRO, black circles). Asterisks indicate significant difference, p<0.05.

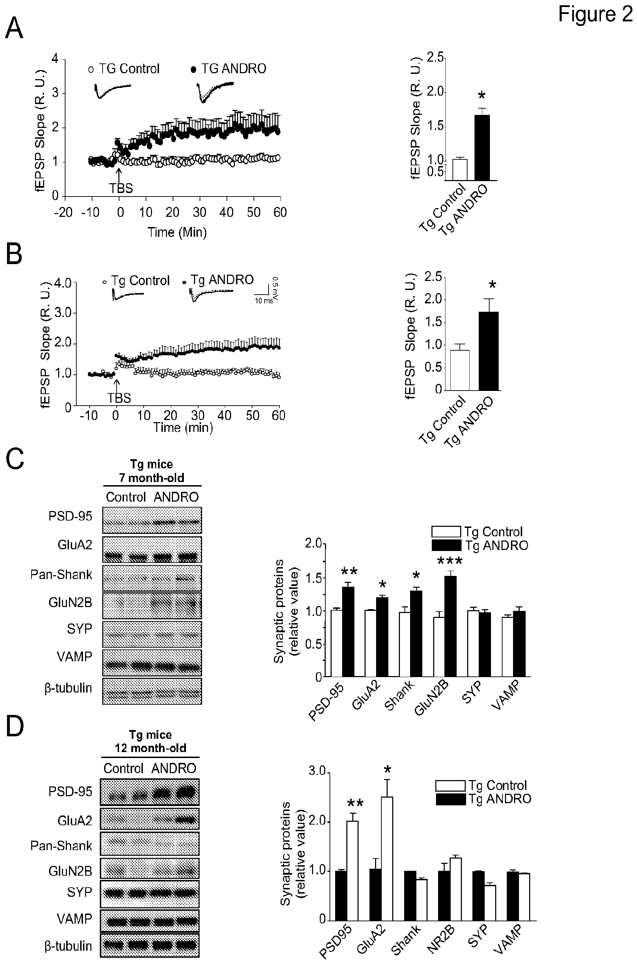

**Figure 2****: ANDRO recovers the LTP induction and synaptic proteins.** (**A**) LTP generated by TBS in hippocampal CA1 in APP/PS 1 mice slices from 12- month-old treated with vehicle solution or ANDRO shows a recovery in the capacity to induce a LTP. (Right panel) Plot of fEPSP slope changes in animals treated after 40 min of recording. The dots and bars are meaning ±SE from 6 different slices, * p< 0.05. (**B**) LTP generated by TBS in hippocampal CA1 in Wt and APP/PS1 mice slices from 7-month-old treated with vehicle solution or ANDRO shows a recovery in the capacity to induce a LTP. (Right panel) Plot of fEPSP slope changes in animals treated after 40 min of recording. The dots and bars are meaning ±SE from 7 different slices, * p< 0.05. (**C, D**) Immunoblots of total protein extracts from hippocampus of Tg Control and Tg ANDRO mice (6-month- and 12-month-old respectively). Graphs correspond to the densitometric analysis of each protein normalized against the loading control and compared to the levels of the same protein in APP/PS 1 control. The loading control in each case was β-tubulin (hippocampus). n≥3, *p<0.05; **p<0.01; *** p<0.001.

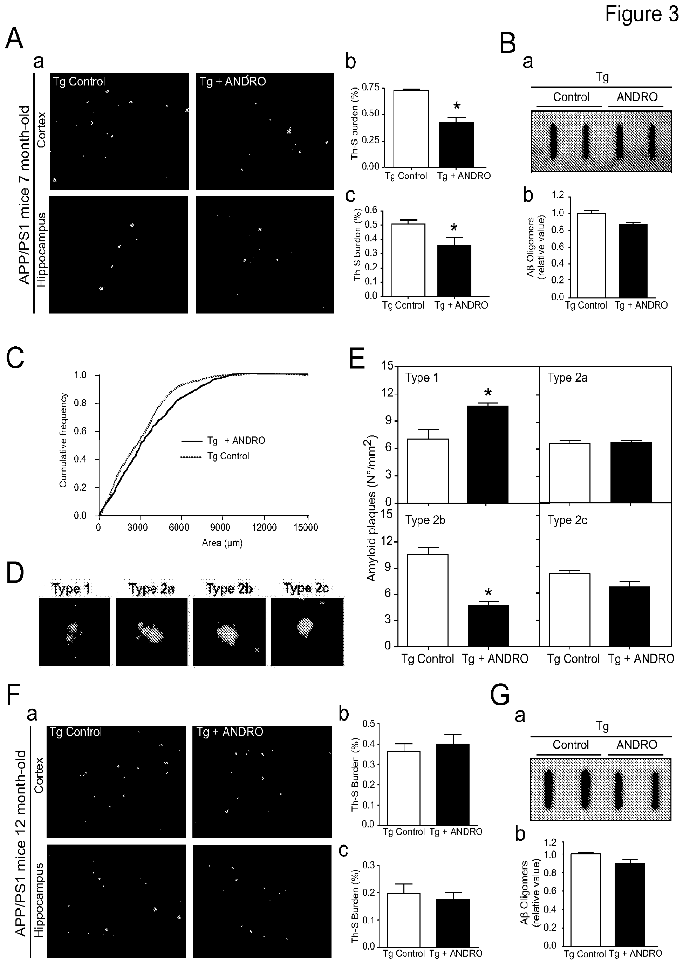

**Figure 3****: ANDRO reduces the Aβ aggregates in brain of young APPswe/PS-1 mice.** (**A, a**) Th-S staining used to detect amyloid deposits of transgenic mice in cortex and hippocampus from 12 month-old transgenic mice with vehicle (Left panel) and ANDRO treatment (Right panel). Amyloid burden was quantified with the Th-S staining and number of amyloid plaques per area in (**a**) cortex and (**b**) hippocampus. White bars show double Tg control mice and black bars show Tg ANDRO, *p<0.05; **p<0.01; *** p<0.001. Picture at 10x. (**B, a**) Representative slot blot from hippocampus, using 6 µg protein per slot spotted into a nitrocellulose membrane and expose to the A11 antibody. (**b**) Graphs represent normalized densitometry analysis profile of the slot intensity for each treatment. (**C**) Cumulative frequency plot of amyloid plaques size for control and ANDRO treated APPswe/PS-1 mice. (**F**) Morphological characterization of the amyloid Th-S positive plaques presented in the brain of young APPswe/PS-1. (**E**) Quantification of the number of each type of amyloid plaque present in the brain. (**Fa**) Th-S staining used to detect amyloid deposits of old (12 month-old) transgenic mice in cortex (**Fb**) and hippocampus (**Fc**) from Tg Control and Tg ANDRO. Amyloid burden was quantified with the Th-S staining. White bars show double Tg control mice and black bars show Tg ANDRO. Picture at 10x. Representative slot blot from hippocampal of old APPswe/PS- 1 mice control and treated with ANDRO (**Ga**), Graphs represent normalized densitometry analysis profile of the slot intensity for each treatment.

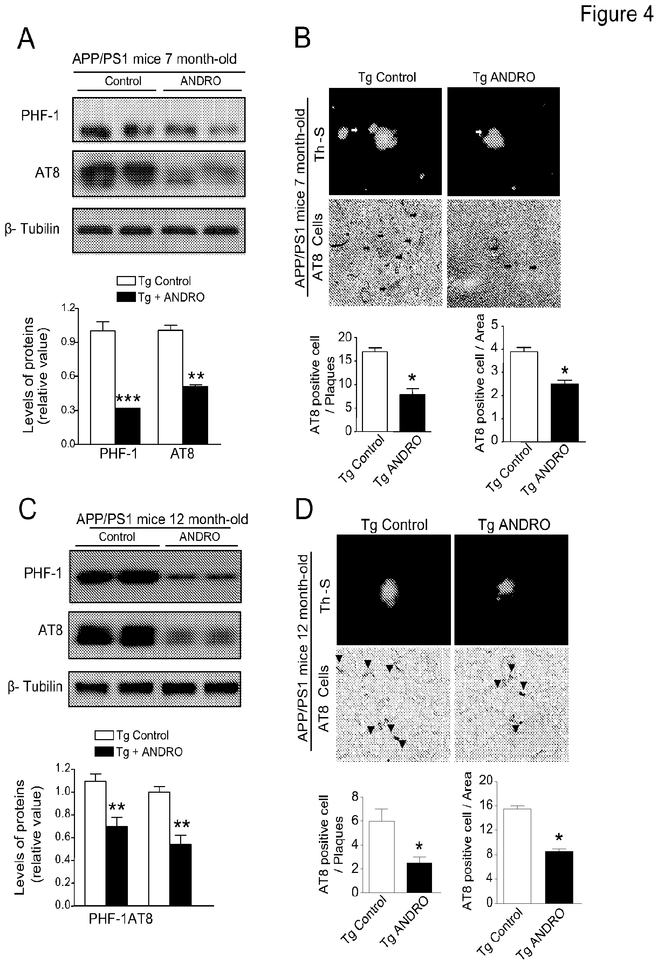

**Figure 4****: ANDRO reduces *tau* phosphorylation in brains of APPswe/PS1 mice from different age. (A)** Immunoblot of hippocampus homogenates from APPswe/PS-1 12 month-old mice (Tg Control and Tg ANDRO) using the PHF-1 and AT8 antibody. (Bottom panel) Graph corresponds to the densitometric analysis of bands normalized against loading control and compared to control APPswe/PS-1 with APPswe treated with ANDRO. **(B)** AT8-positive cells are detected in other regions in APPswe/PS-1 mice control and treated with ANDRO. (Bottom graphs) shows the quantification of the number of AT8-positive neurons per area in mm² and near amyloid plaques detected with ThS. Positive neurons and the amyloid plaques are indicated by black and white arrow respectively. Quantification of the number of AT8-positive neurons per plaque *p<0.05; **p<0.01; *** p<0.001.

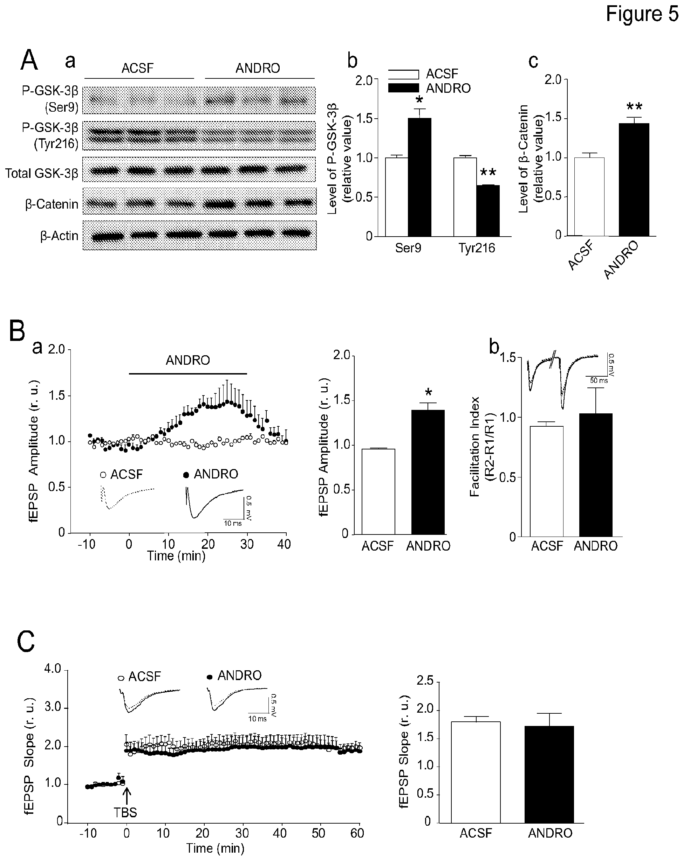

**Figure 5****: ANDRO inhibits GSK-3β allowing the increase in β catenin levels and induce an increase in the synaptic transmission.** Hippocampal slices from Wt animals were exposed to 10 µM of ANDRO for 1 hr. Representative blots of total protein extracts from hippocampal slices treated with ACSF or 10 µM ANDRO. (**A, a**) Treatment with ANDRO for 1h inhibits GSK-3β, this is shown in the increment of Ser-9 phosphorylation (inhibited form of GSK-3β) and in the decrease of Tyr-216 phosphorylation (activated form of GSK-3β). (**A, b**) Graphs of the densitometric analysis of each protein were normalized against total GSK-3β and loading control and compared to the levels of the same protein present in WT control expressed as relative values. (**A, c**) β-catenin was also evaluated in the presence of ANDRO (Right panel). Graphs of the densitometry analysis of each protein normalized against total β-catenin and loading control and compared to the levels of the same protein in WT control expressed as relative value. White bars correspond to ASCF and black bars to ANDRO treated samples. Bars represent the average ± S.E. *p<0.05; **p<0.01 **(B, a)** Plot of fEPSP at different time, in control condition or treated with ANDRO (10 µM). Right panel correspond to Plot of fEPSP amplitude changes, in the presence or absence of ANDRO. **(B, b)** Plot of paired pulse facilitation (PPF) in the presence or absence of ANDRO. Inset shows representative recordings. The dots and bars are mean ±SE from 7 different slices, * p< 0.05. (**C**) Hippocampal slices were exposed to ANDRO and was induced a LTP, the arrow indicates the time of TBS and the plot shows the fEPSP slope at different times. Right panel correspond to Plot of changes in fEPSP slope, in the presence or absence of ANDRO. Inset shows representative recordings. The dots and bars are mean ±SE from 7 different slices, * p< 0.05.

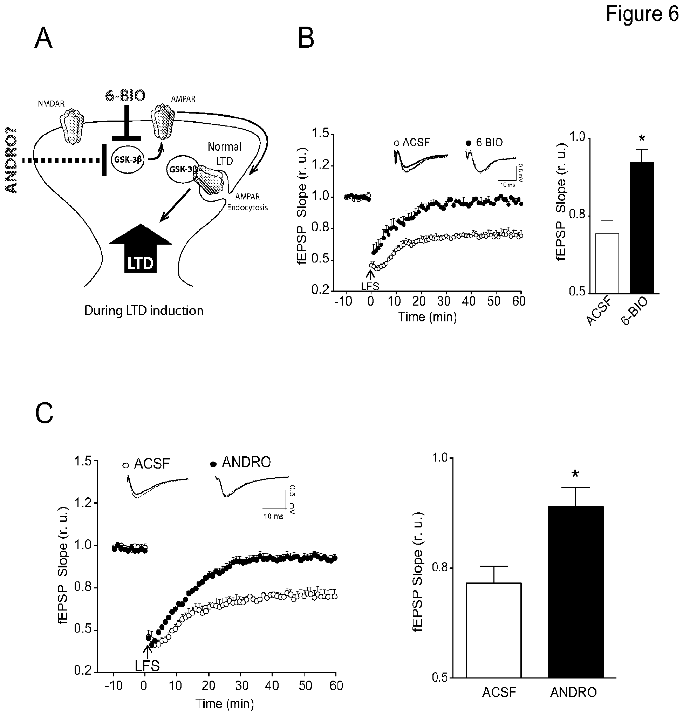

**Figure 6****: ANDRO blocks LTD by GSK-3β inhibition in CA3-CA1 synapse. (A)** Model of Mechanism of action of 6-BIO in the inhibition of GSK-3β during the LTD induction. (**B**) Hippocampal slices were exposed 6-BIO (10 nM) and was induced the LTD, the arrow indicates the time of LFS and the plot shows the fEPSP slope at different times. (Right panel) Plot of changes in fEPSP slope, in the presence or absence of 6-BIO. (**C**) Hippocampal slices were exposed ANDRO and was induced a LTD, the arrow indicates the time of LFS and the plot shows the fEPSP slope at different times. (Right panel) Plot of changes in fEPSP slope, in the presence or absence of ANDRO. Inset shows representative recordings. The dots and bars are mean ±SE from 7 different slices, * p< 0.05.

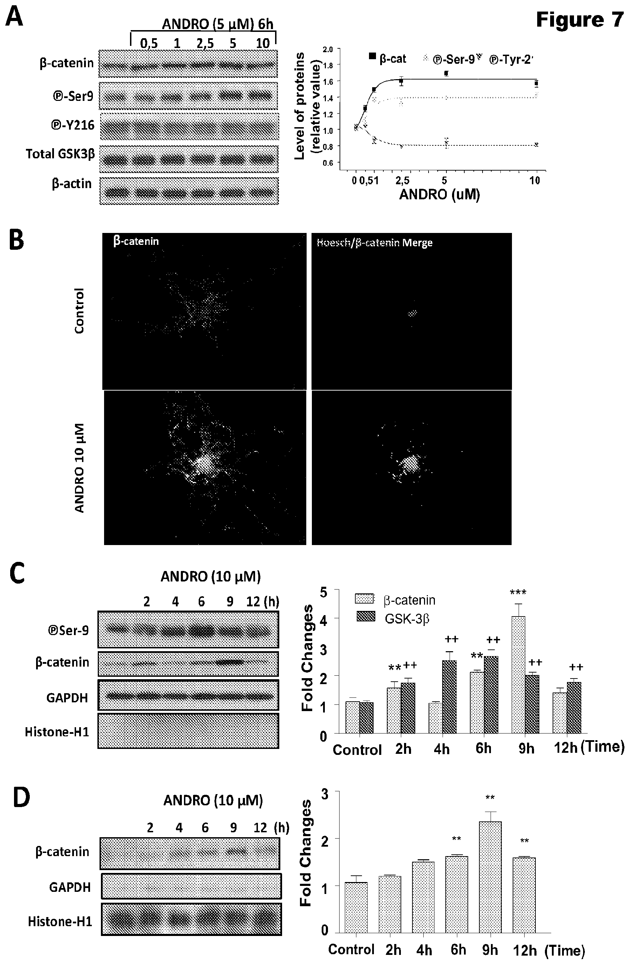

**Figure 7****: ANDRO activate the Wnt canonical pathway. (A)** Representative western blotting of activation Wnt pathway in cultures of hippocampal neurons 14DIV exposed to different concentrations of ANDRO for 6h. **(B)** Immunofluorescence against β-catenin in cultures of hippocampal neurons 14DIV exposed to 5µM ANDRO for 6h. **(C)** Representative western blot of cytoplasmic fraction for timing course of β-catenin and inhibition of GSK-3β, in hippocampal neurons exposed to ANDRO (10 µM). **(D)** Representative western blot of nuclear fraction for timing course of β-catenin, in hippocampal neurons exposed to ANDRO (10 µM). Values represent mean ± S.E (n=4 mice). Student's t-test, ^{*}p<0.05; ^{**}p<0.01.

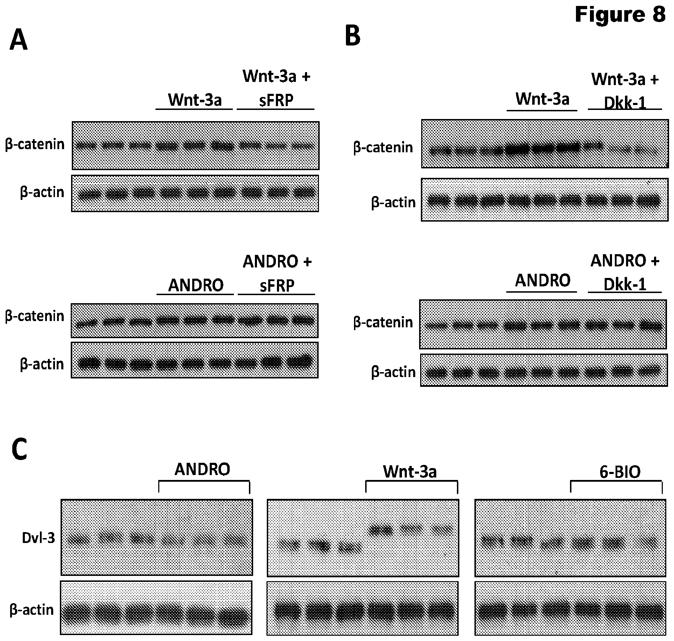

**Figure 8****: ANDRO acts downstream Wnt ligand binding to its receptor. (A)** Slices of hippocampus Co-incubated with ANDRO or conditioned medium Wnt3a in presence of secreted Frizzled-related proteins (sFRP) to analyze the accumulation of β-catenin by western blotting. **(B)** Co-incubation of ANDRO or conditioned medium Wnt3a with the antagonist Wnt Dkk-1 to analyze the accumulation of β-catenin. **(C)** Mobility shift of Dishevelled 3 (Dvl-3) in slices of hippocampus of wild-type two-month old mice in presence of ANDRO, Wnt3a ligand or 6-BIO to analyze the phosphorylation of Dvl-3 by ligand binding to its receptor. Values represent mean ± S.E (n=4 mice). Student's t-test, ^{*}p<0.05; ^{**}p<0.01.

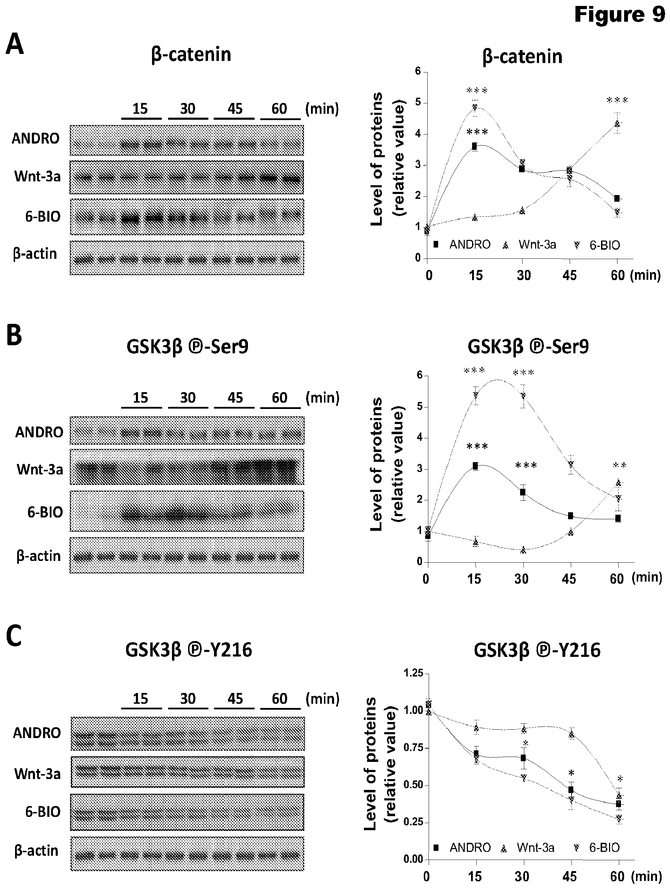

**Figure 9****: ANDRO induces the accumulations of β-catenin and inhibition GSK-3β with similar kinetic of 6BIO. (A)** Timing course of accumulation of β-catenin in presence of ANDRO, conditioned medium Wnt3a or 6-BIO. **(B)** Timing course of inhibition of GSK-3β as indicated for an increment in the phosphorylation of Ser-9 in presence of ANDRO, conditioned medium Wnt3a or 6-BIO. **(C)** Timing course of inhibition of GSK3β as indicated for a decrease in the phosphorylation of Tyr-216 in presence of ANDRO, conditioned medium Wnt3a or 6-BIO. Values represent mean ± S.E (n=4 mice). Student's t-test, ^{*}p<0.05; ^{**}p<0.01.

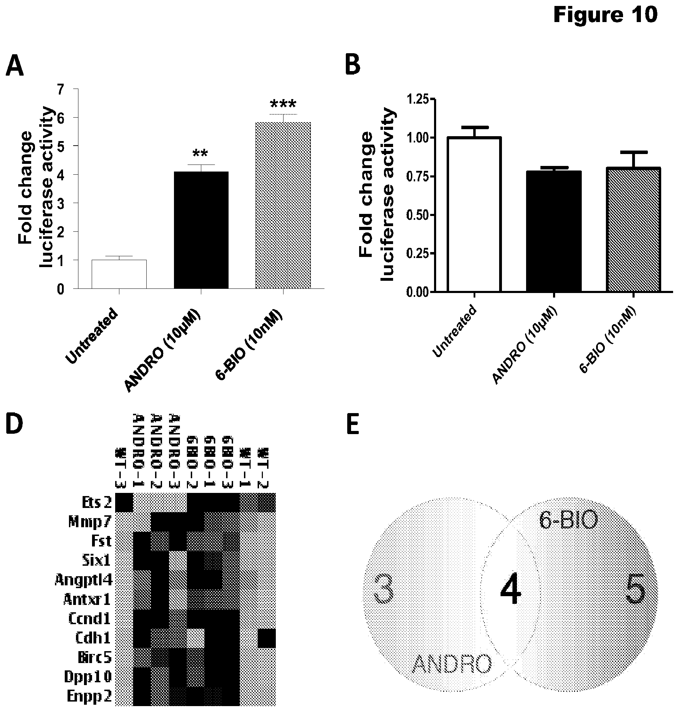

**Figure 10****: ANDRO induces transactivation and transcriptions of Wnt target genes. (A)** TOP flash assay in HEK293T cell for ANDRO or 6BIO. **(B)** FOP flash assay in HEK293T cell for ANDRO or 6-BIO. **(C)** Clustergram of the signal value differentially expressed of Wnt target genes for hippocampal cultures ANDRO and 6-BIO treated for 9 h. **(D)** Venn Diagram of Up-and down regulated Wnt target genes for ANDRO and 6-BIO treatment. Values represent mean ± S.E (n=4 mice). Student's t-test, ^{*}p<0.05; ^{**}p<0.01.

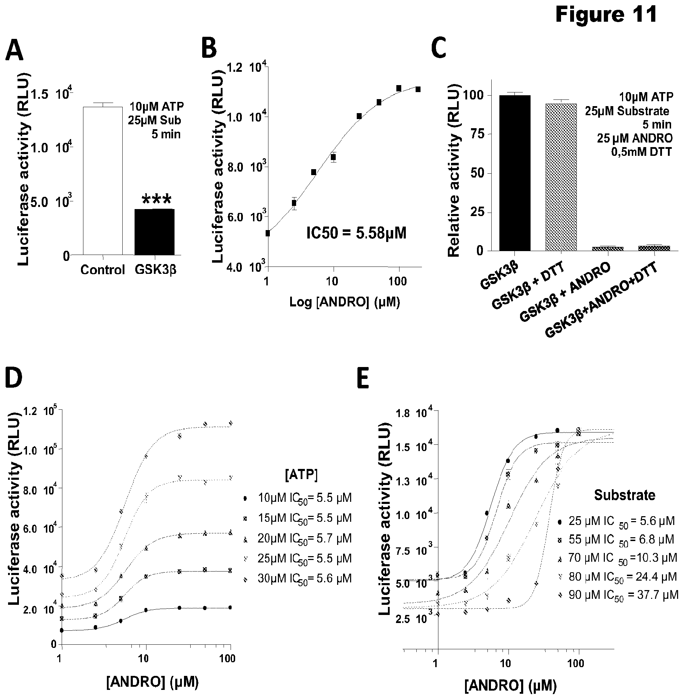

**Figure 11****: ANDRO directly inhibits at GSK-3β competing with substrate binding site (A)** Representative graph of GSK3β activity measure by a luminescence reaction. **(B)** Inhibition assay of GSK3β in presence of different concentrations of ANDRO to determinate the IC50 constant. **(C)** Assay of inhibition in presence or absence of redactor agent DTT to determinate whether the inhibition mechanism of ANDRO implicate a covalent aduct with a cysteine present in the GSK3β. **(D)** Assay of inhibition of ANDRO in presence of different concentrations of ATP and calculation of IC50 values to determinate whether ANDRO is a competitive inhibitor for the ATP binding site. **(E)** Assay of inhibition of ANDRO in presence of different concentrations of substrate and calculation of IC50 values to determinate whether ANDRO is a competitive inhibitor for the substrate binding site. Values represent mean ± S.E (n=4 mice). Student's t-test, ^{*}p<0.05; ^{**}p<0.01.

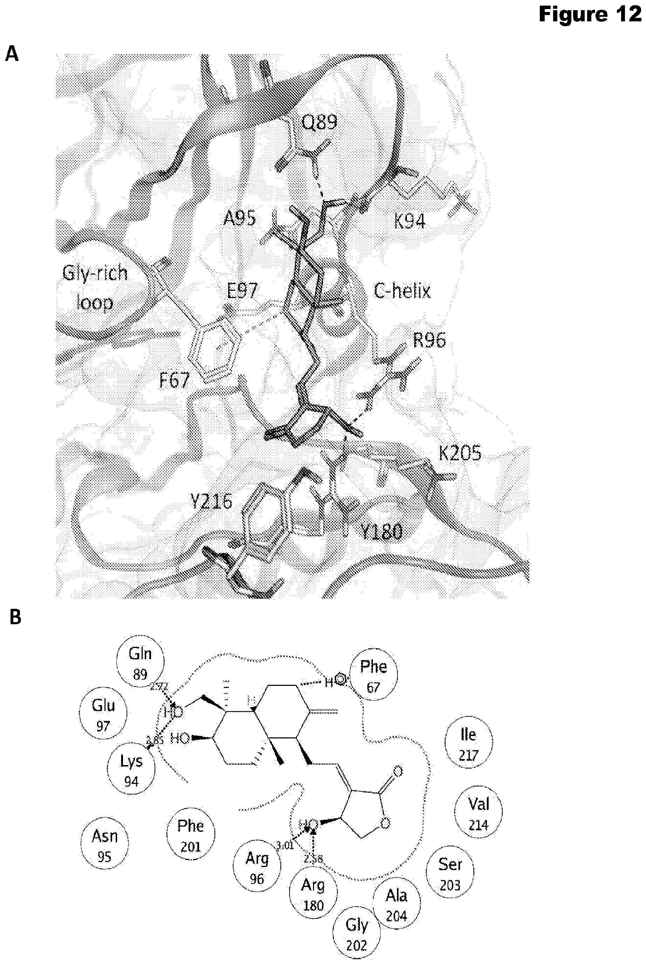

**Figure 12****: Docking of ANDRO and GSK3β in the substrate binding site. (A)** Docking pose of andrographolide (ANDRO) to an area of the substrate binding site of GSK-3β. 20 binding poses were generated with an average ChemPLP score of 51.1 ± 2.2. The ligand is engaged in hydrogen bonding with the priming phosphate binding pocket of GSK-3β (i.e.,Arg-96, Arg-180, Lys-205) and is located within 5 Å of the kinase activation phosphorylation site Tyr-216. **(B)** The docking site if further lined by the residues Phe-67, Gln-89, and Asn-95 which are thought to be involved in substrate recognition.

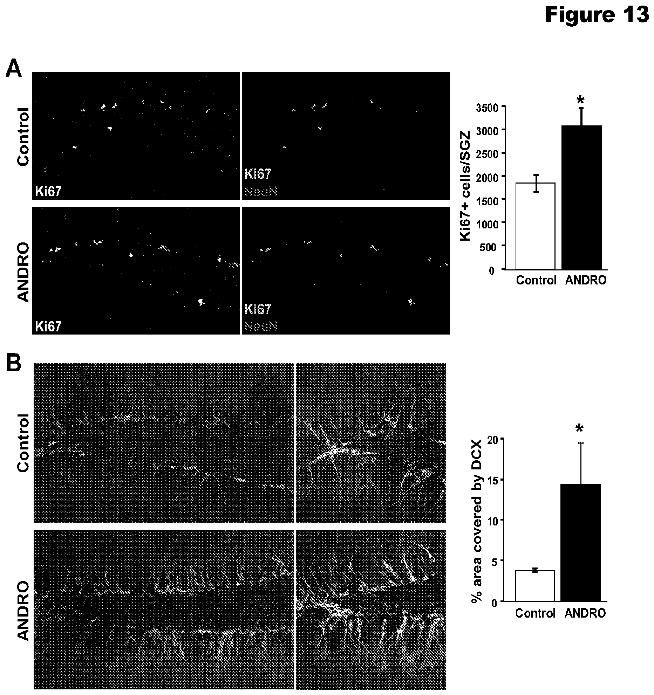

**Figure 13****: ANDRO induces neurogenesis in the hippocampus of adult mice.** Two-month-old wild-type mice were injected i.p. with vehicle alone or vehicle with 2 mg/kg ANDRO 3 times per week for 4 weeks. **(A)** Representative images of the double labeling of Ki67, used to identify proliferating cells, and the mature neuronal marker NeuN in the dentate gyrus of the hippocampus. Images represent projections of z-stacks of 8 optical 1 µm sections. Original Magnification: 20X. A significant increase in the total number of Ki67⁺ cells was observed in mice treated with Andro, indicating that the drug induced cell proliferation. **(B)** Immunofluorescence staining against the immature neuronal marker DCX. Images represent projections of z-stacks of 12 optical 1 µm sections. Original magnification: left panels: 20X; right panels: 40X. A significant increase in the percentage of the granular cell layer area covered by DCX⁺ staining was observed in Andro-treated mice compared to controls. Values represent mean ± S.E (n=4 mice). Student's t-test, ^{*}p<0.05; ^{**}p<0.01.

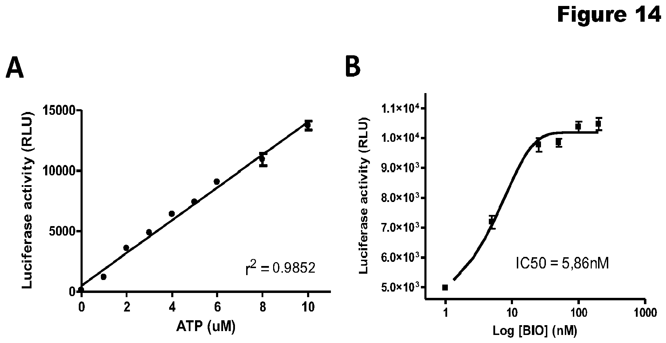

**Figure 14****: (A)** A line graph that compares Luciferase activity (RLU) to ATP (uM). **(B)** A line graph that compares Luciferase activity (RLU) to Log [BIO] (nM).

### DETAILED DESCRIPTION

Our invention is best understood by reviewing our actual experimental results. These results arise from two lines of experiments. In the first (Example 1), we examined the effect of andrographolide on learning and memory behavior in an Alzheimer's model. In the second (Example 2), we examined and quantified certain anatomical changes correlated with andrographolide treatment in an Alzheimer's model.

### EXAMPLE 1

### Material and Methods

**Animals.** Six-month- and Twelve-month-old APPswe/PS-1 double transgenic mice, which express the mutant APPswe (K595N/M596L) and PSEN1ΔE9, the deletion of the exon 9 under the control of the mouse prion promoter, were obtained from the Jackson Laboratory (Bar Harbor, ME, USA, Stock no 004462). The animals were treated and handled according to the National Institutes of Health guidelines (NIH, Baltimore, MD). Treatments were performed by intraperitoneal (IP) injection of 2.0 mg/kg ANDRO with saline as vehicle, three times per week. Transgenic and Wild- type (Wt) control animals were injected only with the vehicle. Control Wt data was pooled after the analyses of the results, since no statistical difference were found between them (data not shown).

**Reagents and antibodies.** Andrographolide (ANDRO, Figure 1A) was obtained from Sigma Chem, (St. Louis, MO). The primary antibodies used were mouse monoclonal anti-PSD-95 (clone K28/43; (Antibodies, Inc, UC Davis/NIH NeuroMab Facility, Davis, CA), mouse anti-Pan Shank (clone N23B/49; UC Davis/NIH NeuroMab), mouse anti-NR2B (clone N59/36; UC Davis/NIH NeuroMab), mouse anti-GluR2 (clone L21/32; UC Davis/NIH NeuroMab), goat anti-synaptophysin (sc-7568 Santa Cruz), rabbit anti- VAMP 1/2/3 (sc-13992 Santa Cruz), mouse anti-*tau*, epitope PHF1 (a kind gift from Dr. Peter Davies, Department of Pathology, Albert Einstein College of Medicine, Bronx, NY), mouse anti-β-Actin clone AC-15 (A1978 Sigma Aldrich), mouse anti-PHF-*tau* clone AT8 (MN 1020, Thermo Scientific), rabbit anti-p44/42 MAPK (Erk1/2) (9102, Cell Signaling), rabbit anti-GSK-3β pSer-9 (Cell Signaling #9336S), anti-GSK-3β pTyr-216 (BD Biosciences #612312), total GSK-3β (sc-9166 Santa Cruz Biotechnology, Inc), mouse anti-Aβ (4G8) and anti-Aβ oligomer (A11) (Chemicon, Temecula, CA)

### Behavioral test.

*Classical model test:* The MWM task was performed as previously described in our laboratory (Braidy et al 2012; De Ferrari et al 2003; Dinamarca et al 2008; Inestrosa et al 2013; Toledo & Inestrosa 2010). Briefly, mice were trained in a circular pool, 1.2 m diameter (opaque water, 50 cm deep) filled with water at 19-21°C. For training, a submerged 9 cm platform (1 cm below the surface of water, invisible to the animal) maximum trial duration 60 sec, 10 sec on platform at the end of trials. Each animal was trained in order to localize the platform, test was performed considering three trials per day and swimming was monitored using an automatic tracking system (HVS Imagen, Hampton, UK). This system was used to measure the latency time needed to reach the platform and the time spent in each quadrant. After testing, the mouse was removed from the maze, dried and returned to its cage.

*Memory flexibility test:* The MWM was performed as previously described in our laboratory (Toledo & Inestrosa 2010). Briefly, mice were trained in a circular water maze of 1.2 m diameter (opaque water, 50 cm deep, 19-21 °C, 9- cm platform 1cm below water, maximum trial duration 60 s, 10 s on platform at the end of trials). Each animal was trained for one pseudo-random location of the platform per day, for 4 days, with a new platform location each day. Training was conducted up to 10 trials per day, until the criterion of 3 successive trials with an escape latency of <20s was met. On completion of testing, the mouse was removed from the maze, dried and returned to its cage. The animals were tested for the next location on the following day. Data were collected using a video tracking system for water maze (HVS Imagen).

**Immunohistochemical procedures.** Perfusion, fixation and free-floating immuno-cytochemical procedures were performed as previously described (De Ferrari et al 2003; Inestrosa et al 2013; Toledo & Inestrosa 2010). Washing and dilution of immune-reagents were performed using 0.01 M PBS with 0.2% Triton X-100 (PBS-T) throughout experiments, with two PBS-T washes per antibody incubation. Sections were pretreated with 0.5% H₂O₂ for 30 min to reduce endogenous peroxidase activity followed by treatment with 3% bovine serum albumin (BSA) at room temperature for 1 h to avoid nonspecific binding. Primary antibodies were incubated overnight at 4°C. Detection of primary antibody was performed using the Pierce ABC Kit (Thermo Fisher Scientific Inc., Rockford, IL). Staining was developed by incubating for 15 min with 0.6% diaminobenzidine followed by the addition of H₂O₂ (0.01% final concentration). After immunostaining, all sections were mounted on gelatin-coated slides, air-dried, dehydrated and cover-slipped with Canada balsam (Merck, Darmstadt, Germany).

The specific antibody used for immunohistochemistry was anti-phosphorylated *tau:* AT8 (1:1000). The sections were pretreated with 0.3% H₂O₂ and then incubated in 3% BSA in PBS. The washes and antibody dilutions were performed using 0.4% Triton X-100 in PBS. Immunohistochemistry was performed using the ABC (avidin biotin-HRP complex) method (Vector Laboratories, Burlingame, CA). Free-floating sections were mounted on gelatin-pre-coated slides, air-dried, dehydrated in graded ethanol and covered with Canada balsam (Merck, Darmstadt, Germany). Image analysis and PHF-1 neuronal counting were carried out as previously described (Cancino et al 2008).

**Thioflavine-S (Th-S) staining.** Th-S staining was developed on gelatin-coated slices as previously described (Carvajal & Inestrosa 2011; Toledo & Inestrosa 2010). After dehydration and rehydration in ethanol and xilol batteries, slices were incubated in distilled water for 10 min and then were immersed in the Th-S solution (0.1% ThS in 70% ethanol) for 5 min. Slices were then washed twice in 70% ethanol for 30 s and cover-slipped with antifade mounting medium in dark.

**Image analysis.** Stained brain sections were photographed using an Olympus BX51 microscope coupled to a Micro-publisher 3.3 RTV camera (QImaging). The luminesce of the incident light and the time of exposure were calibrated to assign pixel values ranging from 0 to 255 in RGB image (no-light to full-light transmission), which were used along all preparations. The images were loaded into ImageJ v.1.40g software (NIH) for analysis. Selection of areas for measurement was performed by manual threshold adjustment or by direct manual selection of ROIs in heterogeneous stains.

**Immunoblotting.** The hippocampus and cortex of treated or control transgenic mice were dissected on ice and immediately processed has detailed previously (Toledo & Inestrosa 2010). Briefly, hippocampal and cortical tissues were homogenized in RIPA buffer (10mM Tris-Cl, pH 7.4, EDTA 5mM, 1% NP-40, 1% sodium deoxycholate, and 1% SDS) supplemented with a protease inhibitor mixture and phosphatase inhibitors (25 mMNaF, 100 mM Na₃VO₄ and 30 µM Na₄P₂0₇) using a Potter homogenizer and then passed sequentially through different caliber syringes. Protein samples were centrifuged at 14000 rpm at 4°C twice for 15 min. Protein concentrations were determined using the BCA Protein Assay Kit (Pierce). Samples were resolved by SDS-PAGE followed by immunoblotting on PVDF membranes. Western blot assays were carried out as previously described (Inestrosa et al 2013; Toledo & Inestrosa 2010).

**Slot Blot.** Slot blots assays were performed, as it was previously described (Toledo & Inestrosa 2010). Briefly, total protein extracts were centrifuged to eliminate fibrillar aggregates at 20000g for 1h. The protein concentration of the soluble fraction was determined and 6 µg of protein were spotted in 0.45 µm² nitrocellulose (Millipore), blocked with PBS-T gelatin 0.4% and incubated with an anti-oligomeric antibody A11 (1/5000) for 2 h at 4°C. Slot blots were revealed with the same methodology as WB.

**Slice Preparation and Electrophysiology.** Hippocampal slices were prepared according to standard procedures previously described (Cerpa et al 2010; VarelaNallar et al 2010). Briefly, transverse slices (350 µm) from the dorsal hippocampus were cut under cold artificial cerebrospinal fluid (ACSF, in mM: 124 NaCl, 2.6 NaHCO3, 10 D-glucose, 2.69 KCl, 1.25 KH2PO4 2.5 CaCl2, 1.3 MgSO4, y 2.60 NaHPO4) using a Vibratome (Leica VT 1000s, Germany) and incubated in ACSF for 1 h at room temperature. In all experiments, 10 µM picrotoxin (PTX) was added to suppress inhibitory GABA**_{A}** transmission (Cuitino et al 2010). Slices were transferred to an experimental chamber (2 ml), superfused (3 ml/min, at 20-22°C) with gassed ACSF and visualized by trans-illumination with a binocular microscope (MSZ-10, Nikon, Melville, NY). To evoke field excitatory postsynaptic potentials (fEPSPs), we stimulate with bipolar concentric electrodes (Platinum/Iridium, 125 µm OD diameter, FHC Inc., Bowdoin, ME) generated by a stimulator (Axon 700b, Molecular Devices, Sunnyvale, CA) and connected to an isolation unit (Isoflex, AMPI, Jerusalem, Israel). The stimulation was in the *Stratum Radiatum* within 100-200 µm from the recording site. The paired pulse facilitation index was calculated by ((R2-R1)/R1), where R1 and R2 were the peak amplitudes of the first and second fEPSP respectively. To generate LTP we used Theta Burst Stimulation (TBS) consisting of 5 trains of stimulus with an inter-train interval of 20 s. Each train consisted of 10 bursts at 5 Hz and each burst having 4 pulses at 100 Hz. To generate LTD we used Low Frequency Stimulation (LFS) consisting in 900 paired pulses at 1 Hz. Recordings were filtered at 2.0-3.0 kHz, sampled at 4.0 kHz using an A/D converter, and stored with pClamp 10 (Molecular Devices). Evoked postsynaptic responses were analyzed off-line, using an analysis software (pClampfit, Molecular Devices), which allowed visual detection of events, computing only those events that exceeded and arbitrary threshold.

**Statistical analysis.** All data are shown as the mean ± SEM, with statistical significance assessed by Student's t-test and ANOVA. All statistical analyses were performed using Prism software (GraphPad Software Inc.).

### Results

### ANDRO recovers the spatial memory in APPswe/PS-1 double transgenic mice from different ages.

Six month- and twelve-month-old set of transgenic APPswe/PS- 1 mice (Garcia-Alloza et al 2006) were treated with ANDRO (Figure 1A) during four weeks. When the treatment concluded, we evaluate the hippocampal function associated with spatial memory by behavioral test task related to learning and memory (Morris et al 1986). Previous evidence indicates that the cognitive deficit associated to spatial memory in young APPswe/PS-1 mice have high variability (Toledo & Inestrosa 2010). Therefore, we evaluate 7-month-old mice performance by a modified spatial memory paradigm associated to episodic memory (memory flexibility) proven to be more sensitive to hippocampal dysfunctions (Chen et al 2000). The analysis of the behavioral performance indicated that Tg animals treated with ANDRO have a reduction in the number of trial to criterion of learning (Figure 1B, gray circles) in comparison with the Tg control (Figure 1B, white circles). On the other hand, we decide to evaluate the effect of ANDRO in 12 month-old APPswe/PS- 1 mice, which is described to have a consistent decrease in cognitive functions. In this aged model, we evaluate by the classical Morris Water Maze (MWM) the spatial memory performance. The analysis of the behavior showed that Wt animals injected with the vehicle have normal escape latencies values during training (Figure 1C, gray circles). In contrast, APPswe/PS-1 had the highest latency values, in agreement with hippocampal dysfunction by neurotoxic effects of A (Toledo & Inestrosa 2010). However, APPswe/PS-1 treated with ANDRO (Figure 1C, black circles) presented significantly lower escape latency values similar to control mice (Figure 1C, white circles) indicating that ANDRO was able to reduce the cognitive impairment in the spatial memory performance during the first and the second week of treatment. These results indicate that ANDRO had beneficial effects against the cognitive impairment present in the APPswe/PS-1 mice in young and mature transgenic mice.

**ANDRO recovers the synaptic functions in the APP/PS1 mice from different ages.**

Consistent with the idea that the improvement of spatial memory is associated with a recovery of synaptic functions in the hippocampus, we evaluated the synaptic plasticity of the two set of APPswe/PS-1 mice by analysis of LTP magnitude in CA3-CA1 transmission in the hippocampus, which also is correlated with the process of memory and learning (Morris et al 1986; Palop & Mucke 2010; Shankar et al 2008). In untreated APPswe/PS-1 mice we do not observed LTP induction, which is consistent with the literature (Palop & Mucke 2010) (Figure 2A white circles). Nevertheless, the two set of APP swe/PS-1 mice treated with ANDRO have the property to induce a LTP that is maintained for 1h (Figure 2A and B, black circles). These results indicate that ANDRO protects the synaptic architecture to allow the induction of synaptic process as LTP in early model of AD and additionally recover the synaptic functions in mature aged APPswe/PS-1 mice.

According with the evidence, the proteins that conform the structures and function of the synaptic transmission and plasticity are diminished in AD brains and altered in transgenic AD models (Scheff et al 2007; Selkoe 2002; Terry et al 1991; Toledo & Inestrosa 2010). Therefore, we carried an analysis of the synaptic proteins affected in APPswe/PS-1 mice in hippocampus and cortex by immunoblotting. As pre-synaptic markers, we evaluated the levels of the synaptic vesicle protein synaptophysin (SYP) and synaptic vesicle-associated integral membrane protein (VAMP). On the other hand, as postsynaptic markers, we evaluated the levels of synaptic scaffold protein Shank, NMDA receptor subunit GluN2B, the AMPA receptor subunit GluA2 and the postsynaptic-density-protein 95 (PSD-95) (Sheng & Kim 2011). As expected in hippocampus and cortex, APP swe/PS-1 mice treated with ANDRO significantly recovers the post-synaptic proteins in young mice (Figure 2C), while the total levels of pre-synaptic proteins SYP and VAMP were unaltered (Figure 2C). Also, we quantified the levels of synaptic proteins in mature Tg mice treated with ANDRO. We observed an increase of PSD-95 and GluA2 levels (Figure 2D), indicating that in models of advanced neurodegeneration still is possible induce a recovery of level of certain proteins associated to process synaptic structure and transmission (Sheng & Kim 2011). These evidences suggest that ANDRO prevents the loss of postsynaptic proteins in 7 month-old mice, but also allow a recovery of important synaptic proteins in mature animals.

### ANDRO decreases the A depositions in young APPswe/PS-1 mice,

Previous studies suggest that in general, the amyloid levels are related to cognitive impairment in AD (Jensen et al 2000). Recent evidence suggest that Aβ oligomers cause the impairment of synaptic functions observed in AD patients and mice models (Shankar & Walsh 2009) and that the targets for Aβ oligomers are mainly located at the postsynaptic region (Lacor et al 2004; Shankar et al 2008; Shankar & Walsh 2009). In order to assess whether the improvement in mice behavior was correlated with a change in AD neuropathological markers, we analyzed the burden of Aβ aggregates present in the brains of young (7 month-old) and old (12 month old) APPswe/PS-1 treated with ANDRO.

We found that in cortex and hippocampus of 7 month-old APPswe/PS-1 mice treated with ANDRO there was a reduction of amyloid plaques stained with Th-S in comparison with untreated animals (Figure 3Aa,b and c). However, the level of Aβ oligomers, evaluated by slot-blot with A11 antibody, in the hippocampus of APPswe/PS-1 mice treated with ANDRO did not show significant changes (Figure 3Ba and b). These results indicate that ANDRO treatment cause a reduction in the overall amount of Aβ plaques, but not in Aβ oligomers (Figure 3Ba, b). A detailed analysis of plaque size distribution was also carried out. The aggregate size distribution presented as a cumulative frequency plot shows that APPswe/PS-1 mice treated with ANDRO has a shift in their distribution toward smaller plaque size (Figure 3C).

To further analyze the effect of ANDRO on amyloid plaques, we performed an analysis of the different amyloid plaques, categorized by maturation stage according to their morphology as follow (Figure 3D) (Bussiere et al 2004): type 1, plaques that have a reticular appearance without a central dense core; type 2, plaques displaying a dense core surrounded by either fibrillar material, in the shape of a corona (type 2a) or radiating from the center (type 2b), or lack any surrounding material (type 2c). The analysis of type 2a, and 2c plaques in the total brain (Fig. 3E) of APPswe/PS-1 mice, treated with ANDRO, showed no significant differences respect to controls. Type 2b plaques, on the other hand, were significantly decreased in the brain of ANDRO-treated APPswe/PS-1 mice. It was observed that most immature plaques (type 1) increase significantly with ANDRO treatment, demonstrating the effect of ANDRO treatment on the maturation of amyloid plaques (Figure 3E).

On the other hand, the analysis of Aβ-aggregates in young APPswe/PS-1 mice is carried out. Transgenic mice showed important levels of amyloid plaques in hippocampus and cortex (Figure 3Fa) and the quantification of the amyloid burden area are shown in Figure 3F (b and c). Mice treated with ANDRO showed no change in amyloid deposit levels in hippocampus or cortex (Figure 3Fb and c). Furthermore, we found that in the hippocampus of APPswe/PS-1 mice treated with ANDRO, the levels of Aβ-oligomers in the hippocampus (Figure 3Ga and b) did not show significant changes.

These results indicate that ANDRO did not affect Aβ load in the old APPswe/PS-1 mouse model but decrease significantly Aβ-aggregates in young transgenic mice treated with ANDRO, suggesting that ANDRO prevent Aβ aggregation in early states during the development of the disease.

### Tau phosphorylation decreases after ANDRO treatment in both APPswe/PS-1 mice.

Another critical hallmark in AD is the *tau* pathology (Mandelkow & Mandelkow 2012) which is characterized by the hyperphosphorylation of *tau* protein that generates the accumulation of paired helical filaments and formation of neurofibrillary tangles (Mandelkow & Mandelkow 2012). In our model, the phosphorylation of *tau* protein in AD epitopes has been described in old animals (Iqbal et al 1989; Jicha et al 1997) and Aβ induces the manifestation of phospho-epitopes in *tau* protein associated with neuronal damage (Vincent et al 1994). Using immunoblotting, we measured *tau* phosphorylation residues associated to AD, that correspond to Ser-396 - Ser-404 (PHF-1) and the residue Ser-202 (AT8) (Figure 4A) (Mercken et al 1992). We found a reduction in PHF-1- and AT8-positive *tau* levels in the hippocampus of 6 month-old APPswe/PS-1 mice treated with ANDRO in comparison with APPswe/PS-1 untreated (Figure 4A and B). Consistent with this idea, reports indicate that the increase of *tau* phosphorylation in specific phosphorylation epitopes surrounding the plaque is a sign of Aβ-induced neuronal damage (Mandelkow & Mandelkow 2012). Accordingly, we study the appearance of the AT8 epitope of *tau* nearby Aβ plaques (Figure 4B). Although *tau* phosphorylation affects different parts of the hippocampus and cortex, we choose to evaluate AT8-positive cells in a circular area (r≈100 mm) surrounding amyloid plaques (Cancino et al 2008), where it has been described that cytoskeletal changes, *tau* phosphorylation, GFAP protein activation and synapse loss occurs (Cancino et al 2008; Selkoe et al 2012). Treatment with ANDRO induced a clear decrease in the number of AT8-positive neurons next to amyloid deposits for 6 month-old APPswe/PS- 1 (Figure 4B, b). The analysis of total AT8-positive cells found outside the circular area around the plaques in the hippocampus showed a decrease in the number of these cells by ANDRO treatment (Figure 4c). These results clearly show that ANDRO is able to prevent *tau* phosphorylation, a key event in AD pathology.

### ANDRO inhibits GSK-3β

The reduction of the phosphorylation of the epitopes of *tau* phosphorylated levels is highly associated to the role of GSK-3β (Mandelkow & Mandelkow 2012), which is one of the enzyme that also modulates the synaptic changes by Aβ and importantly regulate process of plasticity in the hippocampus (Hurtado et al 2012; Peineau et al 2007). Similarly to the previous experiment where evaluated the phosphorylated state of GSK-3β in transgenic animal treated with lithium (Toledo & Inestrosa 2010), we observed that hippocampal slices treated with ANDRO induces a reduction of the active form (Tyr-216) and an increase in the levels of the inactive form of GSK-3β (Ser-9) (Figure 5A). Consistent with this result, we decide to evaluate the activity of GSK-3β in the regulation of certain proteins as β-catenin, a molecule which is phosphorylated by GSK-3β and defines its degradation. We observed an increase in the levels of β-catenin in slices incubated with ANDRO, indicating a decrease in the GSK-3β activity (Figure 5A). This evidence indicates a change in the state of a central enzyme, and possibly in the regulation of the AD. Accordingly, the inactivation of the enzyme could alter the synaptic function and plasticity. Therefore, we explored the effects of ANDRO (5 µM) on the basal synaptic transmission in CA3-CA1 connections in hippocampal slices from Wt animals. Slices exposed to ANDRO for 30 min, increased the fEPSP amplitude (Figure 5B). In order to find whether this effect corresponds to a pre- or postsynaptic effect, the paired pulse facilitation test (PPF) was carried out (Cerpa et al 2010). Results show that ANDRO does not increase PPF ratio (Figure 5B), indicating that the response did not depend of presynaptic changes and probably the response is mediated by an effect in the postsynaptic region. Additionally, GSK-3β do not have a central role in the induction of LTP (Peineau et al 2007), therefore, we evaluated whether ANDRO could be modulate the induction or maintenance of LTP. We do not observed alteration in the LTP induction or maintenance with the presence of ANDRO (Figure 5C) indicating a consistent evidence in the literature about the potential role of GSK-3β (Collingridge et al 2010). This evidence suggests a change in the state of a central enzyme with potential implications in the regulation of the AD.

### ANDRO prevents the induction of LTD

Additionally, the role of GSK-3β is associated with process of synaptic plasticity as the LTD (Bradley et al 2012a; Collingridge et al 2010) where their activation induces the internalization of AMPA receptors from the synaptic spine (Peineau et al 2007). We therefore, explored a possible contribution of ANDRO in the machinery of synaptic plasticity as LTD. First, we used the selective inhibitor of GSK-3β (6-BIO, 10 nM) and studied the role in the induction of LTD (Figure 6A). Note that 6-BIO does not form part of the presently claimed invention.

Consistent with previous evidences, we observed a complete inhibition of the induction of LTD when GSK-3β is inhibited by 6-BIO (Figure 6B).

Consistent with our previous observations, we decided evaluate in the same condition the effect of ANDRO over the induction of LTD. We evaluated in slice treated with ANDRO the change in the magnitude of the fEPSP during LTD induction and we found that the LTD was inhibited with the presence of ANDRO (Figure 6C). The potential role of ANDRO directly or indirectly over GSK-3β indicate a modulation of LTD induction and maintenance. This evidence has potential implications in the development of AD models where it has been described an unbalance in the synaptic plasticity associated with an increase of LTD magnitude over the LTP (Palop & Mucke 2010) and possibly ANDRO could be play a role in the machinery of these synaptic plastic events.

### Discussion

In the present study, we demonstrated that young (7 month-old) and mature (12 month-old) APPswe/PS-1 mice treated with ANDRO recovered cognitive capacities and diminished several neuropathological markers of AD. Specific neuroprotective effects of ANDRO observed in this study include: protection of postsynaptic proteins, reduction of Aβ-oligomeric species and recovery of synaptic functions as LTP in APPswe/PS-1 transgenic mice. Also, ANDRO inhibits the induction of LTD by a mechanism that might include at least the inhibition of GSK-3β activity. Taken together, our data indicate that ANDRO might be of therapeutic relevance in AD.

Other reports indicate the anti-inflammatory effects of ANDRO (Hidalgo et al 2005; Iruretagoyena et al 2005) that can be an additional mechanism responsible for alleviating the late pathological alterations observed in the brain of transgenic mice, and these effects might help in the protective role of ANDRO against the neuroinflammatory response triggered by glial cells (Heneka & O'Banion 2007) protecting the cognitive damage observed in the model of AD (Pike et al 1995). Also, in AD patients there is evidence of the etiopathology of free radicals (Mercken et al 1992; Smith et al 1996), which trigger post-translational modifications in different proteins, including oxidation, glycation and nitrotyrosination, all chemical modifications that stimulate the loss of protein function, as previously described all these effects are particularly relevant in AD (Miranda et al 2000). Several studies report neuroprotection against A mediated cytotoxicity by antioxidants (Dumont & Beal 2011; Quintanilla et al 2005). Therefore, it is possible that ANDRO might possess some antioxidant properties, which play a role in the neuroprotection and prevention of the cognitive capacities observed here in the APPswe/PS-1 transgenic mice treated with ANDRO. Nevertheless, the potential role of ANDRO in process associated to learning and memory observed in AD model treated with ANDRO had not been associated completely to an anti-inflammatory or antioxidant response proposing another mechanism related possibly to synaptic transmission and plasticity. In this context we decided evaluate the synaptic role of ANDRO in the hippocampal CA3-CA1 transmission finding a new relevance of ANDRO in the modulation of the GSK-3β activity with potential implications in the development of AD to different stages.

Recent reports indicate that the interaction between Aβ oligomers and *tau* protein could generate damage and increase the cell death in the brain (Ittner & Gotz 2011). This evidence with respect to the amyloid cascade hypothesis, suggest that Aβ formation is a critical step in driving AD pathogenesis and that it could coexist with the action generated by *tau,* increasing the signal of dysregulation in AD (Selkoe et al 2012). Here, we demonstrate that in APPswe/PS-1 mice treated with ANDRO, *tau* phosphorylation at the PHF-1 and AT-8 sites is reduced, the cognitive function is recovered and the loss in LTP induction and maintenance in CA1 region of hippocampus is reversed. However, we did not see a decrease in the number or size of amyloid plaques in the hippocampus of mature mice indicating that the stable plaques formed are not degraded by ANDRO, and possibly the role of ANDRO could act during or downstream of Aβ aggregation. Additionally, previous reports indicate that the size or numbers of senile plaques are not correlated with cognitive impairment (Lee et al 2004). Apparently, it appears that the Aβ oligomers might play a more relevant role in the synaptotoxicity that altered the cognitive process (Lacor et al 2004). However, we indicate a reduction of the amyloid species in 7-month old transgenic mice, indicating that ANDRO could have a therapeutic relevance in stage of plaque formation before of the stability of the amyloid plaques during the advanced age of the AD model.

These results suggest a functional mechanism of protection triggered by the presence of ANDRO, and this protective property might explain its capacity against cognitive impairment observed in the APPswe/PS-1 mice. In fact, many studies have demonstrated the contribution of hippocampal LTP in memory and learning phenomena, e.g., inhibition or blockade of the proteins that participate in the mechanism as AMPAR or NMDAR subunits negatively modulates spatial memory and behavior (Morris et al 1986; Shankar et al 2008). In models of AD, the impairment of LTP is related to the accumulation of Aβ oligomers, which trigger postsynaptic failures, loss of synaptic proteins and finally a loss of spatial memory and learning (Haass & Selkoe 2007; Shankar & Walsh 2009). The ANDRO effect shows protection of LTP and postsynaptic proteins and this might be another relevant factor in the restoration of the cognitive impairment at an early stage of the disease. Interestingly, the treatment of ANDRO in different stages in the AD model protect from the reduction of synaptic proteins inclusive with the not alteration of Aβ oligomers in the transgenic mice. These could indicate that the presence of ANDRO could have a potential effect in the signaling of Aβ oligomers during changes in the synaptic transmission and plasticity.

Reports indicate a pathway related to the cascade generated by Aβ oligomers that activate proteins such as caspase-3, and a non-apoptotic pathway that induces the inhibition of Akt1, which in turn contributes to the over activation of GSK-3β (Hurtado et al 2012; Jo et al 2011). These events induce the internalization of AMPA receptors from the dendrite spines facilitating the induction of LTD (Bradley et al 2012b; Jo et al 2011; Peineau et al 2007; Shankar et al 2008), which occurs before plaque formation and neuronal cell death. Such effects are triggered by Aβ oligomers, which are soluble and toxic molecular forms of Aβ (Cerpa et al 2008; Haass & Selkoe 2007; Mucke & Selkoe 2012; Shankar et al 2008). These pathways are involved in the development of the synaptic failures in AD models (Palop & Mucke 2010) and treatment with inhibitors or shRNAs that specifically block GSK-3β induced a phenotype similar to those described for the AD model treated with ANDRO (Hurtado et al 2012; Jo et al 2011; Peineau et al 2007; Toledo & Inestrosa 2010). Regarding the possible mechanisms by which ANDRO might exert its effect at the postsynaptic site, we found that ANDRO inhibits LTD by inactivation of GSK-3β, preventing the mechanism of action of the Aβ oligomers. Also, the inhibition of GSK-3β may implicate the activation of different pathways associated with survival and protection of the neurons. In this context, *Wnt* signaling is activated after the inhibition of GSK-3β by lithium and this phenomenon protects against cognitive impairment (Toledo & Inestrosa 2010). In fact, we showed an increase in the βcatenin levels with ANDRO, and recent studies from our lab indicate that ANDRO induces a strong activation of the canonical *Wnt* signaling pathway (Tapia-Rojas et al., unpublished results). In summary, we have identified a natural drug that inhibits GSK-3β and might be blocking the LTD observed in the CA1 region of the hippocampus in the presence of Aβ oligomers, allowing the recovery of cognitive function. The absence of effective drugs or treatments available for AD allows new possibilities to obtain natural products with selective effects in AD models, that might permit an alternative approach to obtain new therapeutics to treat and prevent the progression of neurodegenerative diseases, including AD.

### EXAMPLE 2

### Andrographolide activates Wnt/β-catenin signaling by direct inhibition of GSK-3β in hippocampal cells and stimulates adult neurogenesis in vivo.

### Introduction

The Wnt/β-catenin signaling pathway regulates multiple biological processes and its dysfunction is associated with a number of diseases. The use of natural compounds is an interesting strategy to search for potential drugs that activate this pathway and may have a therapeutic potential. Here we report that Andrographolide (ANDRO), a component of *Andrographis paniculata,* is a potent activator of the Wnt signaling. ANDRO activates this pathway by a mechanism that bypasses the Wnt receptor and involves inhibition of GSK-3β. *In silico* analyses suggest that ANDRO interacts with the GSK-3β substrate-binding pocket, which was tested demonstrating that ANDRO competes for site. Then, we evaluated whether ANDRO was able to regulate an *in vivo* process regulated by the Wnt signaling pathway, adult hippocampal neurogenesis. Treatment of 2 month-old wild-type mice with ANDRO for 4 weeks strongly induces cell proliferation and the generation of newborn neurons in the dentate gyrus of the hippocampus. Altogether, our results indicate that ANDRO is an *in vivo* activator of the Wnt signaling pathway that could be used as a therapeutic strategy for the treatment of diseases associated to a Wnt signaling impairment and in pathological conditions in which there is a decreased neurogenesis.

Glycogen synthase kinase-3β (GSK-3β) is an enzyme discovered about 30 years ago. This kinase phosphorylates substrates with a recognition sequence Ser/Thr-X-X-X-pSer/pThr, and the prior action of a "priming" kinase is critical to catalyze the phosphorylation (Dajani et al, 2001; Frame et al, 2001). GSK-3β activity is regulated by phosphorylation within their amino-terminal domain; phosphorylation in Ser-9 residue inhibits the enzyme while the phosphorylation of Tyr-216 within the T-loop region, modification that appears to be the result of an autophosphorylation reaction, enhanced the activity of the enzyme (Cole et al, 2004; Hughes et al, 1993). Although GSK-3β was originally identified as a kinase involved in the regulation of glycogen metabolism, it is now known to participate in the control of many cellular processes including embryonic development, where it is a key component of the Wnt signaling pathway, as well as gene transcription and neuronal cell function (Hoeflich et al, 2000; Inestrosa & Arenas, 2010; Jiang et al, 2005; McManus et al, 2005). The Wnt pathway has been described as an essential signaling in cell adhesion and regulation of cell fate determination during development (Patapoutian & Reichardt, 2000), and in the nervous system it participates in the neural tube formation and midbrain development. The canonical or Wnt/βcatenin signaling pathway is activated by the interaction of a Wnt ligand with a receptor member of the Frizzled (Fz) family and co-receptor LRP5/6 (refs). This binding triggers the phosphorylation of Dishevelled (Dvl), which in turn inactivate GSK-3β, a key modulator of this signaling pathway (Gao & Chen, 2010). As result, the intracellular levels of β-catenin increase, allowing its import to the nucleus where it binds to members of the family of T-cell factor/lymphoid enhancer-binding factor (TCF/LEF) transcription factors and activates the expression of Wnt target genes (Clevers & Nusse, 2012) that contains a consensus sequence in its promoters, called Wnt Response Element (WRE) (Mosimann et al, 2009). Conversely, in the absence of a Wnt ligand, GSK-3β forms a high-molecular weight complex that includes Axin, the adenomatous poliposis coli (APC) component and β-catenin, its activity is switched on and phosphorylates β-catenin which is targeted for ubiquitination and subsequent degradation by the proteasome (Aberle et al, 1997).

The Wnt signaling pathway has relevant roles in the adult nervous system (Inestrosa and Arenas, 2010), and dysfunction of this pathway is associated with several mental diseases, including schizophrenia, mood disorders, autism, and Alzheimer's disease (AD) (Balu & Lucki, 2009). Neurogenesis is one of the processes regulated by the Wnt signaling pathway in the adult brain. The Wnt/β-catenin pathway is active in the subgranular zone (SGZ) of the hippocampus (Lie et al,2005), which is one of the neurogenic regions in the adult brain, and it regulates progenitor cells proliferation and neuronal differentiation. The *in vivo* block of the Wnt pathway in the hippocampus reduced neurogenesis and impaired learning and memory (Lie 2005; Jessberger).

A negative relationship between aging and neurogenesis has been described (Kuhn et al, 1996; Luo et al, 2006; Olariu et al, 2007). An 80% reduction in neurogenesis in the dentate gyrus of aged mice compared to young adults has been reported (Cameron & McKay, 1999; Kuhn et al, 1996; Olariu et al, 2007). Also, a decreased neurogenesis is observed in different pathological conditions. Neural stem cell proliferation was decreased in the hippocampus of patients with schizophrenia, and it has been suggested that this may contribute to the pathogenesis of the disease (Reif et al, 2006). Work in rodent models of depressed mood showed suppressed hippocampal neurogenesis while pharmacotherapeutic antidepressants elevated neurogenesis (Winner et al, 2011). Also, an impaired neurogenesis has been reported in different transgenic mouse models of AD (Lazarov & Marr, 2010 ; Marlatt & Lucassen, 2010).

The emerging role of Wnt signaling as a therapeutic target for neurodegenerative diseases led us to evaluate potential drugs able to activate this signaling pathway. One component of the Wnt signaling pathway that may be an interesting target for the design of novel therapeutic interventions is GSK-3β. *Andrographis paniculata* contains high amounts of andrographolide (ANDRO) (Cheung et al, 2001; Lu et al, 1981; Panossian et al, 2000) and has been traditionally used as a natural remedy for the treatment of inflammatory process so as to treat laryngitis, diarrhea and rheumatoid arthritis in China, India, Korea and Japan. However, little is known about other pharmacological target related to its known anti-inflammatory actions (Chen et al, 2011; Suebsasana et al, 2009; Xia et al, 2004). In diverse studies it has been suggested that ANDRO might exert some neuroprotective effects. Using a rat model of cerebral ischaemia it was observed that ANDRO induced suppression of NF-kB and a reduction in the production of cytokines and pro-inflammatory factors (Chan et al, 2010). It was also reported that ANDRO treatment exerts protective effects against nicotine toxicity and mitochondrial oxidative stress in different brain regions (Das et al, 2009). Likewise, ANDRO possess a role in antiplatelet activity, which involves the activation of the eNOSNO/cyclic GMP pathway, resulting in the inhibition of the PI3 kinase/Akt-p38 MAPK and PLCy2PKC cascades (Lu et al, 2011), important signaling pathways involved in apoptosis and that could crosstalk with GSK-3β signaling.

In the present work, we analyzed the effects of ANDRO on the activation of Wnt signaling. Our results show that ANDRO activates the canonical Wnt pathway with the consequent transcription of Wnt target genes with a different kinetic to the one reported for the canonical ligand Wnt-3a. The effects of ANDRO were not repressed by the presence of inhibitors of the Wnt signaling like dickopf-1 (Dkk-1) and soluble Fz related protein (sFRP), both acting at the receptor level. Moreover, Dvl-3 was not phosphorylated by the action of ANDRO suggesting a downstream action of the drug. Activity assays show that ANDRO directly inhibits GSK-3β competing by the substrate binding site, as is also suggested by *in silico* studies of molecular docking. Finally, *in vivo* treatment with ANDRO induced cell proliferation and increased the generation of new neurons in the dentate gyrus, indicating that ANDRO increases neurogenesis in the adult hippocampus. These results suggest that *in vivo* ANDRO stimulates the Wnt/β-catenin pathway by inhibition of GSK-3β indicating that this drug could be used as a potential treatment for diseases related to Wnt signaling dysfunction and neurodegenerative disease.

### Materials and Methods

**Reagents and antibodies.** Andrographolide (ANDRO) was obtained from Sigma Chem, (St. Louis, MO). GSK-3β inhibitor 6-bromoindirubin-3'-oxime (BIO) was purchased from Sigma (#B1686). The primary antibodies used were mouse anti-β-Actin clone AC-15 (A1978 Sigma Aldrich), rabbit anti-GSK-3β pSer-9 (Cell Signaling #9336S), anti-GSK-3β pTyr-216 (BD Biosciences #612312), total GSK-3β (sc-9166 Santa Cruz Biotechnology, Inc), anti-β-catenin (H-102) (sc-7199 Santa Cruz Biotechnology, Inc), anti-GAPDH (sc-7199 Santa Cruz Biotechnology, Inc)

**Slice preparation.** Hippocampal slices from two-month-old wild-type animals were prepared according to standard procedures (Varela-Nallar et al, 2010). Transverse slices (250-300 µm) from the dorsal hippocampus were cut under oxygen-saturated cold artificial cerebrospinal fluid using a Vibroslice microtome (VSL, World Precision Instruments, Inc WPI, Sarasota, FL). After obtained, slices were incubated in ACSF for at least than one hour at room temperature, in order to stabilize activity. In every moment during dissection, slicing and experimentation slices were oxygenated with 95% oxygen-5% carbon dioxide.

**Primary culture of rat hippocampal neurons.** Rat hippocampal cultures were prepared as previously described (Arrazola et al, 2009). On day 3 of culture, hippocampal cells were treated with 2mM 1-b-Darabinofuranosylcytosine (AraC) during 24 h to reduce the number of proliferating non-neuronal cells. This method resulted in highly enriched cultures for neurons (5% glia). **PCR-ARRAY.** RNA from hippocampal neurons was extracted using Trizol. Quantification and purity of RNA preparations were checked by absorbance using Nanodrop ND-2000. Only preparations with a 260/280 nm ratio ≥ 1.8, a 260/230 nm ratio ≥ 1,7and no sign of RNA degradations, verified by agarose gel electrophoresis with Gel Red TM were used. Genomic DNA was degraded. Using RT2First-Strand cDNA Synthesis kit (SuperArray), 1µg total RNA was reverse transcribed and then real-time PCR was performed by adding cDNA directly to PCR Master Mix, containing SYBR Green and ROX. The mixtures were then aliquoted into a 96-well PCR array plates, which profiles the expression of 84 Wnt target genes from rat. The PCR was run on ABI StepOnePlus, with 1 cycle of 10min at 95°C followed by 40 cycles of 15s at 95°C and 1min at 60°C. Cycle threshold (Ct) values were calculated for all the genes present on the array (Schmittgen & Livak, 2008).

**Treatment of neuronal cultures.** Primary hippocampal cultures of neurons 14 DIV were washed twice in neurobasal medium and maintained for 1 hour. Next ANDRO (10 µM) and 6-BIO (10 nM) were added to different time periods.

**Nuclear and cytoplasmic extraction.** Following a time-course treatment, neurons were washed with cold PBS and harvested in 600µE of cold homogenization buffer (10mM HEPES pH 7,9; 1,5mM MgCl2; 10mM KCl; 1mM DTT). The cells were disrupted by 20 passed, back and forth, through a syringe of 1mL. Lysates were centrifugedfor6 minutes at 700g at 4°C and the supernatant was removed. The precipitate was washed in 100µL of buffer (20mM HEPES pH=7,9; 420mM NaCl; 1,5mM MgCl2; 0,2mM EDTA; 1mM DTT; 25% glycerol) and centrifuged again to generate nuclear fraction. Cytosolic fractions were generated from the supernatant 100.000g for 1hat 4 °C. Both buffers were supplemented with protease inhibitors and serine-phosphatase. Nuclear and cytosolic fractions were subsequently subjected to western blotting.

**Luciferase Assay.** 150,000 HEK293T cells were seeded in triplicate per condition and kept for 24 hours at 37°C with 5% CO2. The cultures were transfected with TOPflash or FOPflash (400 ng), which are firefly-luciferase reporter DNA constructs (Millipore), using LipofectAMINE 2000 (Invitrogen). To control for transfection pRL-TK (10 ng) was included in all transfections. ANDRO (10 µM) or 6-BIO (10nM) was added to the culture 24 h after transfection. After 24 h the medium was removed from cells, washed with PBS and the firefly and renilla luciferase activities were measured using Dual-Luciferase Reporter Assay System (Promega) with a Luminometer TURNER DESIGNS model TD-2020. Values were normalized by the renilla value from the same well. To statistically compare treatments ANOVA was used.

**Immunoblotting.** The samples were dissected on ice and immediately frozen in liquid nitrogen or processed has previously detailed (Inestrosa et al, 2011). Briefly, tissues were homogenized in RIPA buffer (10mM Tris-Cl, pH 7.4, EDTA 5mM, 1% NP-40, 1% sodium deoxycholate, and 1% SDS) supplemented with a protease inhibitor mixture (1 mM PMSF, 2 µg/mL aprotinin, 1 µg/mL pepstatin and 10 µg/mL benzamidine) and phosphatase inhibitors (25 mMNaF, 100 mM Na3VO4, 1 mM EDTA and 30 µM Na4P207) using a Potter homogenizer and then passed sequentially through different caliber syringes. Protein samples were centrifuged at 14000 rpm at 4° C twice for 15 min. Protein concentration was determined using the BCA Protein Assay Kit (Pierce). Samples were resolved by 12% SDS-PAGE were resolved by 10% SDS-PAGE and transferred to a PVDF membrane. The reactions were followed by incubation with anti-mouse, anti-goat or anti-rabbit IgG peroxidase conjugated (Pierce, Rockford, IL) and developed using an ECL kit (Western Lightning Plus ECL, PerkinElmer).

**Immunoprecipitation.** The immunoprecipitation was performed as previously described (Garrido et al, 2002). Briefly, the hippocampus of two month-old wild-type mice were lysed in immunoprecipitation buffer (25 mM HEPES pH 7.4, 125 mMNaCl, 25 mM NaF, 1 mM EDTA, 1 mM EGTA, 1% NP-40) including phosphatase inhibitors (1 mM NaVO3 and 200 nM okadaic acid) and the protease inhibitor cocktail described above. 200 µg of total proteins from lysate were precleared by using protein G-Sepharose beads (Santa Cruz Biotechnology) for 1 h at 4°C and then were incubated with anti-GSK-3β antibody (Santa Cruz Biotechnology) and new protein G beads for 4 h at 4°C. The immunocomplexes were finally pelleted and washed three times with cold immunoprecipitation buffer.

**GSK-3β activity assay.** IC50 of ANDRO was determined by a luminometric GSK-3β activity assay. This method has been previously described and gives comparable IC50 values to radioactive detection (Baki et al, 2007). Briefly, ANDRO was tested at different concentrations and diluted in kinase buffer containing final concentrations of: 40mM Tris pH 7.5; 20mM MgCl2; 0,1mg/ml BSA. Diluted ANDRO was added to 25 µM pGS-2 peptide substrate (Millipore, Billerica, USA), immunoprecipitated GSK-3β and 10 µM ATP (Cell Signaling, Boston, USA) to a total assay volume of 50µl. The enzymatic reaction was stopped after 10 min of incubation to room temperature by adding 50µl Kinase Glo (Promega, Madison, USA). The luminometric signal was allowed to stabilize for 10 min and the measured with a Luminometer TURNER DESIGNS model TD-2020.

**Molecular docking.** 29 conformers were generated for andrographolide with the LowModeMD search method (MMFF94x force field) of the Molecular Operating Environment (MOE) version 2010.10 (Chemical Computing Group, Montreal, Canada) to explore alternative ring conformations. The LowModeMD method employs short molecular dynamics simulations utilizing velocities with little kinetic energy on the high-frequency vibrational modes (Labute, 2010). The lowest energy conformer was selected for docking. Next, the crystal structure of GSK-3β in complex with 6-BIO (PDB entry 1uv5) (Meijer et al, 2003) was prepared for docking with MOE. Hydrogen atoms were added and the complex was subjected to restrained energy minimization (AMBER99 force field and generalized Born solvation model) until the RMS gradient fell below 0.05 kcal (molÅ)-1. Water molecules and the co-crystalized ligand 6-BIO were removed. Molecular docking was performed with GOLD version 5.1 (The Cambridge Crystallographic Data Centre, Cambridge, UK) (Jones & Willett, 1995). Possible binding sites for andrographolide were explored by docking to the entire protein. Then residues within 11Å from the sulfur of Cys-199 were defined as the ATP binding pocket and atoms within 12Å from the backbone nitrogen of Ser-203were defined as the substrate binding pocket. 20 docking poses were generated with the CHEMPLP scoring function and automatic search parameters (200% efficiency).

**Immunofluorescence.** Hippocampal neurons were seeded onto poly-L-lysine-coated cover slips in 24-well culture plates at a density of 5x10⁴ cells per well, as described previously (Arrazola et al, 2009; Chacon et al, 2008). A mouse monoclonal anti-b-catenin was used. Cells were extensively washed with PBS and then incubated with Alexa 488-conjugated anti-mouse IgG for 1 h at room temperature. Cells were mounted in mounting medium and analyzed in a Carl-Zeiss Confocal Laser Microscope.

**Animals.** Two-month-old C57BC6 wild-type mice were treated and handled according to National Institutes of Health guidelines (NIH, Baltimore, MD). The treatments were performed by intraperitoneal (ip) injection of 2.0 mg/kg ANDRO and wild-type control animals were injected only with saline solution as vehicle.

**Perfusion and post-fixation.** Mice were anesthetized (100 µg ketamine + 10 µg xylazine in 10 µl saline/g), and then transcardially perfused with saline, followed by 4% paraformaldehyde (PFA) in 0.1M PBS. The brains were removed and incubated inPBS-4% PFA for 24 h at room temperature, dehydrated in 30% sucrose and kept at 4°C until analysis.

**Tissue sectioning.** After dehydration, each mouse brain was sectioned on a cryostate in 12 sets of serial coronal sections of 40µm thickness (Leica Microsystems, Wetzlar, Germany) and collected in ice-cold-PBS in multiwell dishes. Each set contained 5-7 sections covering the entire length of the hippocampus.

**Immunofluorescence for Ki67 and Doublecortin (DCX).** Sections were washed with PBS 3 times for 5 min and then incubated in blocking solution (3% donkey serum, 3% BSA, 0.5% Triton X-100 in PBS) for 4 h. Then, tissues were incubated overnight with primary antibodies at 4°C. Primary antibodies used were: rabbit anti-Ki67 (Abcam), rabbit anti-DCX (Cell Signaling Technology Inc., Beverly, MA, USA), monoclonal anti-NeuN (Millipore, Billerica, MA, USA). After washing 3 times with 0.5% Triton X-100 PBS, sections were incubated with Dy Light (Abcam) conjugated secondary antibodies for 2 h at room temperature. Slices were mounted on gelatin-coated slides with Fluoromont-G(Electron Microscopy Sciences, Hatfield, PA, USA). For quantification, Ki67 positive cells were counted using a fluorescence microscope (Olympus BX51, Tokyo, Japan). Cells were counted in all sections of one set (see tissue sectioning). Total cells counted in one set were multiplied by the total number of sets to obtain an estimation of the total number of Ki67+ cells in the complete hippocampus. For quantification of the % area covered by DCX, double labeling DCX/NeuN was carried. The total area of the granular cell layer was estimated as the area positive for NeuN staining and the percentage of this area covered by DCX staining was estimated. This image analysis was carried out using the ImageJ software (NIH, USA). Images shown were analyzed by confocal laser microscopy (Olympus FV 1000) and z-projections were generated with ImageJ software.

**Statistical analysis.** All data are shown as the mean ± SEM, with statistical significance assessed by One-way ANOVA with post test Bonferroni. All statistical analyses were performed using Prism software (GraphPad Software Inc.).

### Results

### ANDRO activates the Wnt/β-catenin pathway in cultured rat hippocampal neurons.

To assess if ANDRO is able to modulate the Wnt/β-catenin pathway in neurons, hippocampal neurons in culture were treated with ANDRO at different concentrations for 6 h. As shown in Figure 7A, ANDRO inhibits GSK-3β as indicated by the increase in the inhibited form of the enzyme (phospho-Ser-9) and the decrease in the active form (phosphor-Tyr-216), which correlate with an accumulation of β-catenin in a concentration-dependent manner. β-catenin is an integral structural component of cadherin-based adherent junctions, and a key effector of the canonical Wnt pathway in the nucleus (Valenta et al, 2012). As assessed by immunofluorescence staining, treatment with 5 µM ANDRO for 6 h increased β-catenin levels in cultured hippocampal neurons at 14 days *in vitro* (DIV) (Figure 7B). Wnt signaling activation triggers the stabilization of β-catenin in the cytoplasm and its translocation into the nucleus where it activates the transcriptional activation of the Wnt target genes (Nusse, 2012). We evaluated cytoplasmic and nuclear levels of β-catenin in neurons treated with ANDRO, and observed a peak accumulation in at 9 h of treatment (Figure 7C and 7D). Interestingly, the maximum accumulation of β-catenin is preceded by the maximum increase of the inhibited form of GSK-3β at 6 h (Figure 7C). These results suggest that ANDRO activates the canonical Wnt/β-catenin signaling pathway.

### ANDRO activates the Wnt/β-catenin pathway by a mechanism independent of the ligand-receptor binding.

Wnt signaling antagonists can inhibit the pathway activation by either binding to the Wnt receptors or co-receptors or by direct interaction with Wnt ligands preventing its action. The Dickkopf (Dkk) family of secreted proteins acts as antagonists of canonical Wnt signaling by either binding to LRP5/6 and preventing it from interacting with Wnt-Frizzled complexes, or by binding to Kremen proteins triggering the internalization of LRP-5/6 (Caraci et al, 2008; Niehrs, 2006). Other antagonists of Wnts are the members of the sFRPs family which antagonizes Wnt signaling by directly binding to Wnt ligands through their N-terminal cysteine-rich domains (CRDs), preventing its binding to Fz receptors (Bovolenta et al, 2008; Mii & Taira, 2011). The incubation with sFRP (Figure 8A) and Dkk-1 (Figure 8B) for 15 min prior to the treatment with 5 µM ANDRO or Wnt3a conditioned medium for 1 h shows that these Wnt antagonists are able to inhibit the accumulation of β-catenin in the presence of Wnt3a but not in presence of ANDRO, suggesting that the drug targets components downstream Wnt receptors.

To further investigate this hypothesis, we evaluate the phosphorylation of Dvl-3 which phosphorylation is triggered by the binding of a Wnt ligand to a Fz receptors and to co-receptors (Gao & Chen, 2010). Hippocampal slices were incubated with ANDRO for 1 h and the phosphorylation of Dvl was evaluated by the mobility shift through western blot. Figure 8C shows that contrary to what was observed by Wnt-3a treatment, ANDRO does not induce a mobility shift of Dvl-3. As expected, no mobility shift in Dvl was observed by treatment with 6-bromoindirubin3'-oxime (6-BIO), a direct and selective inhibitor of GSK-3β competitive for the ATP binding site (Figure 7C). Altogether, these results suggest that the target of ANDRO responsible for the stabilization of β-catenin may be downstream the Dvl.

Lithium and another compound that inhibits the enzyme GSK-3β produce an increase in intracellular β-catenin levels and to the consequent activation of the Wnt/β-catenin pathway (Toledo & Inestrosa, 2010). In order to analyze the temporality of the stabilization of β-catenin, hippocampal slices from 2 month-old wild-type mice were exposed to 5µM ANDRO, conditioned medium of Wnt3a ligand or 10 nM 6-BIO, for different time periods until 1 h. Figure 9A shows that ANDRO as well as 6-BIO increased total levels of β-catenin at 15 min of treatment, while the effect of the canonical Wnt-3a ligand was observed at 45 min. In the same manner, the increase in the levels of the inactive GSK3β phosphorylated at Ser-9 (Figure 9B) and decrease in the levels of the active GSK-3β Tyr-216 phosphorylated (Figure 9C) was faster in ANDRO and 6-BIO treatments compared to Wnt-3a treatment. These results suggest that ANDRO activates the canonical Wnt pathway by a different mechanism than Wnt-3a ligand, and support a receptor-independent mechanism.

### ANDRO induces the transcription of Wnt target genes.

Wnt signaling culminates with the translocation of β-catenin to the nucleus and the transcriptional activation of Wnt target genes (Jamieson et al, 2012). The possible transactivating ability of ANDRO treatment was analyzed with the TOP/FOP luciferase reporter system. The TOPflash reporter contains the TCF/LEF consensus sequence DNA binding sites upstream of a luciferase reporter gene, while FOPflash contains these sites muted. ANDRO treatment as well 6-BIO caused a significant increase in the TOPflash (Figure 10A) but not in the FOPflash (Figure 10B) reporter activity, suggesting that ANDRO may specifically induce the transcription of WRE coupled genes. To study the effect of ANDRO in gene expression, we used a platform that contains numerous Wnt target genes expressed in diverse cell types of rat. We incubated cultured hippocampal neurons with ANDRO or 6-BIO for 9 h (Figure 10D), the time of higher accumulation of nuclear β-catenin by ANDRO treatment (Figure 7C). Of the 86 genes analyzed, only 12 showed a change. Surprisingly only 4 of them were shared by both ANDRO and 6-BIO, while 3 and 4 genes were specifically modulated by ANDRO and 6-BIO respectively (Figures 10D and 10E). These results show that ANDRO is able to induce the transcription of Wnt target genes.

### ANDRO directly inhibits GSK-3β by competing with the substrate binding site

Considering the effects of ANDRO on β-catenin levels and activation of Wnt target genes, and considering that the mechanism seems to be downstream the receptors and Dvl, we evaluated the possibility that ANDRO may directly inhibit GSK-3β. The search for GSK-3 inhibitors is a very active research field for both academic centers and pharmaceutical companies because GSK-3β has been shown to have key roles in regulating a diverse range of cellular functions as well as a target for diabetes and neurodegenerative diseases (Cohen & Goedert, 2004). To determinate whether ANDRO is able to directly inhibit GSK-3β activity assays were performed. Immunoprecipitated GSK-3β from hippocampus of wild-type mice was incubated with ATP and pGS-2 peptide substrate for 5 min, then the reaction was stopped and the kinase activity was determined in a luminometer.

The luminescence was directly proportional to the concentration of ATP (Figure 14A). Figure 11A shows the GSK-3β activity, follows a decrease in the luciferase activity by ATP consumption in presence of kinase. In Figure 11B GSK-3β was previously incubated with different concentrations of ANDRO for 10 min. The graph shows that ANDRO inhibits the activity of the kinase with an inhibition constant (IC50) of 5.58 µM. In order to validate the inhibition assay, we calculated the IC50 for 6-BIO (Figure 14B) which agrees with the one previously reported (5nM) (Meijer et al, 2003; Polychronopoulos et al, 2004).

Previous studies regarding the pharmacological mechanism of ANDRO and its known anti-inflammatory actions demonstrate that ANDRO covalently conjugates reduced cysteine 62 of p50, thus preventing NF-κB oligonucleotide binding to p50, inhibiting nuclear NF-κB transcriptional activity, and attenuating inflammation in various *in vitro* assays and *in vivo* models (Xia et al, 2004; Yao et al, 2012). To evaluate whether ANDRO is able to inhibit GSK-3β by a covalent conjugate with a cysteine residue present in the kinase, we incubated ANDRO in the presence of 0.5 mM DTT with GSK-3β previous at activity assay. The results show that the presence of DTT does not affect the kinase activity neither prevents the inhibition generated by ANDRO (Figure 11C), indicating that the mechanism of ANDRO does not involve the formation of a covalent adduct between a molecule of GSK-3β and a molecule of ANDRO. 6-BIO is a potent, reversible and ATP-competitive GSK3β inhibitor, therefore we evaluated whether ANDRO could inhibit GSK-3β competing for the ATP binding site. We calculated the IC50 values of ANDRO in the presence of different concentrations of ATP (Figure 11D). The IC50 of ANDRO using different concentrations of ATP was very closed to the calculated 5.58 µM, indicating that the inhibition mechanism does not involve competence for the ATP binding site. To determinate the possibility that ANDRO inhibit GSK-3β by competing with the substrate binding site, we calculated the IC50 values in the presence of different concentrations of the substrate (Figure 11E). There was an increase in the IC50 of ANDRO when the substrate concentration was increased, which indicates that ANDRO is a competitive inhibitor of GSK-3β that competes for the substrate binding site of the enzyme.

### In silico analyses suggest interaction of ANDRO with the GSK-3β substrate binding site

The results of the GSK-3β inhibition assays are indicative of a (non-ATP) substrate competitive mode of inhibition. To identify a potential binding site of ANDRO we docked the compound with the software GOLD to the three-dimensional crystal structure of GSK-3β. Two areas comprising the ATP and the substrate binding sites were initially identified as potential binding pockets with comparable docking scores for ANDRO (ATP binding site ChemPLP score: 52.5 ± 1.4; substrate binding site ChemPLP score: 51.1 ± 2.2). Interaction with the ATP binding site seemed unlikely, since the predicted docking poses did not form any hydrogen bonds with the hinge region of the kinase, an essential feature observed in known ATP-competitive inhibitors. However, docking poses generated in the substrate binding site showed interactions with residues important for the substrate binding. In Figure 12 we show a representative docking pose of ANDRO bound to the substrate binding site of human GSK-3β co-crystallized with the ATP-competitive inhibitor 6-BIO (PDB entry 1uv5). However, due to structural flexibility, a number of additional ANDRO binding geometries were found for different structural conformers of GSK-3β. To account for structural flexibility we docked ANDRO to thirty-seven human GSK-3β crystal structures available from the Protein Databank (see Materials and Methods). Kinase residues contacted in more than 50% of all docking poses include Phe-67, Leu-88, Gly-90, Val-93, Lys-94, Asn-95, Arg-96, and Gly-202. Of these, Arg-96 together with Arg-180 and Lys-205 form a positively charged oxyanion binding site which was suggested to accommodate the P+4 phospho-Ser/Thr sidechain of primed substrate peptides. Interactions with Arg-96 were observed in 95% of all docking poses, and in 16% with Arg-180. Blocking the oxyanion site could readily interfere with phosphorylation of primed substrates. Alanine mutation of Arg-96 abolished kinase activity towards primed substrates. Additionally, Phe-67 (75% contacts), Asn-95 (96% contacts) and Gln-89 (23% contacts) were shown to be important in substrate recognition *in vitro* and *in vivo.* Mutation of these residues to alanine significantly decreased phosphorylation of several substrates, including β-catenin. Blocking the binding pocket lined by Phe-67, Gln-89, Asn-95 could prevent access of substrate peptides to the kinase and thus inhibits substrate phosphorylation. In summary, the *in silico* analysis predicted interaction of ANDRO with residues of the substrate binding site important for specific recognition and phosphorylation of GSK-3β substrates.

### ANDRO induces cell proliferation and neurogenesis in the hippocampal dentate gyrus.

Finally, we evaluated the effect of ANDRO on hippocampal neurogenesis as an *in vivo* process that is positively regulated by the Wnt signaling pathway. The effect of ANDRO on cell proliferation was evaluated in the SGZ of 2-month-old mice injected i.p. with 2 mg/kg ANDRO or with vehicle solution as a control (saline), 3 times per week for 4 weeks. Ki67 staining (Figure 13A, left panels) was used as a mitotic marker (Kee et al, 2002). Treatment with ANDRO significantly increased the total number of Ki67⁺ cells in the SGZ indicating that ANDRO stimulates cell proliferation at the dentate gyrus. In addition to the increase in proliferation, ANDRO treatment increased the generation of new neurons as determined by an increase in the density of immature neurons positive for the immature neuronal marker DCX (Figure 13B). In fact, the percentage of the granular cell layer covered by DCX staining was significantly increased in the hippocampus of mice treated with ANDRO for 4 weeks (Figure 13B, graphic). Interestingly, DCX⁺ cells show a more complex morphology in mice treated with ANDRO compared to control mice (Figure 13B, right panels), suggesting that the drug also induced the development of newborn neurons. Altogether, these findings indicate that ANDRO increases neurogenesis in the adult hippocampus.

### Discussion

In this study, we showed that *in vitro* ANDRO treatment induces the stabilization of β-catenin and induces the Wnt target genes by a mechanism that bypasses the ligand/receptor binding and implicates the direct inhibition of GSK-3β competing by the substrate binding site. In vivo, ANDRO treatment increases the proliferation and generation of new born neurons in the dentate gyrus of the hippocampus of adult mice. These results point to ANDRO as a potential drug for the treatment of diseases related to Wnt signaling dysfunction as well as to pathologies that involve adult neurogenesis.

ANDRO is a potent anticancer and anti-inflammatory agent isolated from the plant *Andrographis paniculata.* It has been reported to be cytotoxic against diverse cancer cell lines *in vitro* and also acting as a potent anti-inflammatory by interfering directly with NF-κB protein (Xia et al, 2004). Here we show a new and interest target to ANDRO: The inhibition of GSK-3β. GSK-3 was originally identified in 1980 and is highly conserved in diverse species from yeast to mammals (Embi et al, 1980). Mammals express two GSK-3 isoforms, α (51kDa) and β (47 kDa), that are encoded by distinct genes and its amino acid is identical in a 97% within their catalytic domains. However, their sequences differ significantly outside the kinase domain (Wada, 2009). Despite the specificity given by its name it is a central and key regulatory kinase in a wide range of biological processes and has multiple substrates as part of different signaling cascades (Grimes & Jope, 2001). Both GSK-3 isoforms are expressed ubiquitously and although both isoforms are highly similar in structure and sequence, it is clear that GSK-3α cannot compensate all the effects of GSK-3β e.g. GSK3β -/- mice die in utero due to hepatocyte apoptosis (Hoeflich et al, 2000). Several studies have associated the deregulation of GSK-3β with a number of pathological disorders, including diabetes mellitus, obesity, inflammation, bipolar disorder, neurodegenerative disease and cancer (Rayasam et al, 2009; Wada, 2009; Woodgett, 2003).

GSK-3β was first implicated in Wnt signaling owing to the induction of a dorsal ventral axis duplication phenotype by its dominant negative form in Xenopus laevis embryos (Dominguez et al, 1995; He et al, 1995) that now is associated with Wnt-β-catenin signaling pathway activation. Subsequently, β-catenin was identified as a GSK-3β substrate and GSK3-mediated phosphorylation triggers β-catenin destabilization and degradation by the proteasome (Aberle et al, 1997; Yost et al, 1996). This finding established GSK-3β as a key component in the canonical Wnt signaling cascade. Later on, new roles for GSK-3β in this signaling pathway, besides the phosphorylation of β-catenin, were identified. Among these, it was established that GSK-3β phosphorylates the Wnt co-receptor low density lipoprotein receptor-related protein LRP5 or LRP6 (Wu & Pan, 2009).

Wnt signaling plays an important role in regulating cell proliferation and differentiation during development and in the adult hippocampus is a regulator of synapse structure and function (Inestrosa & Arenas, 2010). Deregulation of the Wnt pathway has been implicated in many human diseases, including diverse types of cancer, pulmonary fibrosis and skeletal disorders (Luo et al, 2007). Wnt signaling is strongly regulated at multiple cellular levels therefore it seems feasible to therapeutically modulate the pathway at diverse points. Considering the fundamental role of GSK3β in the signaling cascade, the activity of this enzyme has been a very attractive therapeutic target, the design or discovery of highly specific and efficient inhibitors of GSK-3β that does not have side effects, is a appealing.

We identified ANDRO as an inhibitor of GSK-3β competing by substrate binding site. To demonstrate the involvement of this kinase in a biological process, signaling pathway or to treat patients with any associated to hyperactivation pathology is necessary posses an inhibitor higher selective. At present more than 30 GSK-3 inhibitors have been identified and some with IC50 values in the nanomolar range. However, the most widely used inhibitors, such as SB216763 or 6BIO, compete for the ATP-binding pockets of the enzyme. Because GSK-3α and GSK-3β are very similar structurally, inhibitors that target the ATP-binding sites are unlikely to differentiate between the two isoforms. According to our results, ANDRO may act as an isoform selective inhibitor because of its inhibition mechanism involves the competition for the substrate binding site. However, further experiments will be required to determinate whether ANDRO is an isoform-specific inhibitor of GSK-3β.

ANDRO induced accumulation of β-catenin in both cytoplasmic and nuclear fractions in hippocampal neurons suggesting it may activate the transcription of Wnt target genes, possibility that was assessed in HEK293T cells. This cell line was selected because these cells: i) have a high transfection rate; ii) have been used for the TOPflash reporter assays system (Killick et al, 2001; Hooper et al, 2011); (iii) show endogenous levels of TCP1, LEF1, TCF4 and TCF3 sufficient for β-catenin-dependent transactivation (Klymkowsky et al, 1999). ANDRO increased luciferase reporter 4-fold while the effect of 6-BIO was almost 6-fold over control the condition. This suggest different transactivating abilities of these two inhibitors of GSK-3β. In addition, we observed differences in the effect of these inhibitors on the expression of Wnt target genes. Interestingly, a recent study showed that GSK-3 inhibition by two distinct pathways such as insulin and Wnt3a (much faster) causes a differential expression of seven genes (Hooper et al, 2011).

In our results, after a Bonferroni correction for multiple samples, was determined the down-regulation of Ets2 gene by ANDRO treatment. Interestingly, this gene has been up-regulated in Down's syndrome and in AD patients to median age (Coyle et al, 1986) and over-expression studies of this gene in human cortical neurons have determined a series of changes that lead to apoptotic neuronal death (Helguera et al, 2005). Another gene that presented differential expression induced by ANDRO and does not by 6-BIO is Cdh-1, which is associated with neuronal apoptosis in the hippocampus after global cerebral ischemia (Zhang et al, 2011). GSK-3β is a key mediator of apoptosis (Turenne & Price, 2001) and thereby might directly contribute to neuronal loss in neurodegenerative disorders so that its inhibition is an important event preventing neuronal death. Our results indicate that ANDRO induces the expression of survival genes and shows another therapeutic approach to this natural compound.

Also, we observed that ANDRO treatment increased cell proliferation and neurogenesis in the dentate gyrus of the hippocampus. The SGZ of the dentate gyrus is one of the regions where there is a continuous generation of new neurons in the adult brain (Gage, 2000). In the SGZ, radial and non-radial neural precursor cells give rise to transient amplifying progenitors that generate neuroblasts and then became immature neurons that mature and integrate into the preexisting hippocampal circuitry (vanPraag et al, 2002). Many signaling pathways have been identified as regulators of this process (Ming & Song, 2011; Suh et al, 2009), and Wnts are among the signaling molecules of the niche that regulate neurogenesis. It has been determined *in vivo* that blocking the Wnt signaling decreases adult hippocampal neurogenesis while stimulating this pathway has the opposite effect (Lie et al, 2005). Wnt-mediated effects in neurogenesis are caused by the transcriptional activation of proneural genes (Karalay et al, 2011; Kuwabara et al, 2009). Therefore, it is likely that ANDRO induces cell proliferation and the generation of newborn neurons in the hippocampus by its inhibitory effect on GSK-3β and the consequence modulation of Wnt target gene transcription. Importantly, the effect on neurogenesis indicates that ANDRO treatment is effective *in vivo.*

In summary, this study shows that ANDRO is a new competitive inhibitor of GSK-3β in neurons which seems not to cause side effects in animals, suggesting this may be an effective drug for the treatment of pathologies associated to Wnt signaling dysfunction such as AD.

### ANDROGRAPHOLIDE

Our murine experiments used andrographolide purchased from Sigma-Aldrich Chemical Company.

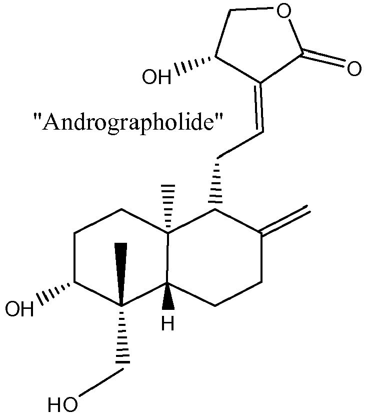

Andrographolide has CAS Registry No. 5508-58-7 and systematic name 3-(2-(Decahydro-6-hydroxy-5-(hydroxymethyl)-5,8a-dimethyl-2-methylenenaphthyl)ethylidene)dihydro-4-hydroxyfuran-2(3H)-one.

### SUMMARY

Our research shows that andrographolide produces a constellation of distinct anatomical changes in the brain of a murine model for Alzheimer's disease. These anatomical changes are correlated with distinct behavioral changes: we found that andrographolide treatment enabled Alzheimer's model test subjects to perform as well as normal healthy subjects on a swimming test of short-term learning and memory. We found that andrographolide induces a reduction in the active state of Glycogen Synthase Kinase 3β, blocking the Long-Term Depression in the CA1 region of the hippocampus, allowing the recovery of cognitive function in an Alzheimer's disease model from different ages.

While our actual testing used pure andrographolide as sold by Sigma-Aldrich Chemical Co., one could alternatively use a plant extract containing an effective amount of andrographolide, or any of various pharmaceutically-acceptable salts of andrographolide.

## Claims

1. Andrographolide for use in a method for the treatment of Alzheimer's disease dementia in a human wherein andrographolide is administered in a therapeutically effective amount as a daily oral dosage of from 1 to 4 milligrams per kilogram of body weight of a human.

2. Andrographolide for use according to claim 1, wherein the method for the treatment of Alzheimer's disease dementia in a human comprises:
a) Administering to a human a test selected from the group consisting of: the Mini Mental Status Exam (MMSE), the Alzheimer Disease Assessment Scale (ADAS), the Boston Naming Test (BNT) and the Token Test (TT); and then
b) Administering andrographolide to said human, for at least 30 days, a daily dosage effective to preserve or improve cognitive function.

3. Andrographolide for use according to claim 1 or claim 2, wherein andrographolide is provided in a therapeutically effective amount.

4. Andrographolide for use according to any one of claims 1 to 3, wherein andrographolide is provided as an extract of *Andrographis paniculata,* standardized to contain not less than 50% (w/w) of andrographolide.

5. Andrographolide for use according to any one of claims 1 to 3, wherein andrographolide is pure andrographolide.

## Patentansprüche

1. Andrographolid zur Verwendung in einem Verfahren für die Behandlung von Morbus Alzheimer-Demenz bei einem Menschen, wobei Andrographolid in einer therapeutisch wirksamen Menge als orale Tagesdosis von 1 bis 4 Milligramm pro Kilogramm Körpergewicht eines Menschen verabreicht wird.

2. Andrographolid zur Verwendung nach Anspruch 1, wobei das Verfahren für die Behandlung von Morbus Alzheimer-Demenz bei einem Menschen Folgendes umfasst:
a) Verabreichen eines Tests, ausgewählt aus der Gruppe bestehend aus: Mini Mental Status Exam (MMSE), Alzheimer Disease Assessment Scale (ADAS), Boston Naming Test (BNT) und Token Test (TT), an einen Menschen; und anschließend
b) Verabreichen von Andrographolid an den Menschen für mindestens 30 Tage eine Tagesdosis, die wirksam ist, um die kognitive Funktion aufrechtzuerhalten oder zu verbessern.

3. Andrographolid zur Verwendung nach Anspruch 1 oder Anspruch 2, wobei Andrographolid in einer therapeutisch wirksamen Menge verabreicht wird.

4. Andrographolid zur Verwendung nach einem der Ansprüche 1 bis 3, wobei Andrographolid als Extrakt von *Andrographis paniculata* bereitgestellt wird, der so standardisiert wurde, dass er nicht weniger als 50% (w/w) Andrographolid enthält.

5. Andrographolid zur Verwendung nach einem der Ansprüche 1 bis 3, wobei es sich bei dem Andrographolid um reines Andrographolid handelt.

## Revendications

1. Andrographolide pour utilisation dans une méthode de traitement d'une démence de type maladie d'Alzheimer chez un humain, où l'andrographolide est administré dans une quantité thérapeutiquement active forme d'une dose orale quotidienne comprise entre 1 et 4 milligrammes par kilogramme de poids corporel d'un humain.

2. Andrographolide pour utilisation selon la revendication 1, où la méthode de traitement de la démence de type maladie d'Alzheimer chez un humain comprend :
a) l'administration à un humain d'un test choisi dans le groupe constitué par : MMSE (Mini Mental Status Exam), ADAS (Alzheimer Disease Assessment Scale), BNT (Boston Naming Test) et TT (Token Test) ; puis
b) l'administration d'andrographolide audit humain, pendant au moins 30 jours, une dose quotidienne suffisante pour conserver ou améliorer les fonctions cognitives.

3. Andrographolide pour utilisation selon la revendication 1 ou la revendication 2, où l'andrographolide est fourni dans une quantité thérapeutiquement active.

4. Andrographolide pour utilisation selon l'une quelconque des revendications 1 à 3, où l'andrographolide est fourni sous forme d'un extrait d'*Andrographis paniculata,* normalisé pour contenir au minimum 50 % en masse d'andrographolide.

5. Andrographolide pour utilisation selon l'une quelconque des revendications 1 à 3, où l'andrographolide est de l'andrographolide pur.
